(19) **Europäisches Patentamt**

**European Patent Office**

**Office européen des brevets**

(11) **EP 1 024 761 B1**

(12) **EUROPEAN PATENT SPECIFICATION**

(45) Date of publication and mention
of the grant of the patent:
**14.08.2002 Bulletin 2002/33**

(51) Int Cl.⁷: $A61B\ 18/12$

(86) International application number:
**PCT/US98/21357**

(21) Application number: **98953352.6**

(22) Date of filing: **09.10.1998**

(87) International publication number:
**WO 99/18878 (22.04.1999 Gazette 1999/16)**

(54) **SOFT TISSUE COAGULATION PROBE**

WEICHGEWEBEKOAGULATIONSSONDE

SONDE DE COAGULATION POUR TISSUS MOUS

(84) Designated Contracting States:
**DE ES FR GB IT NL SE**

(30) Priority: **10.10.1997 US 949117**
       **10.10.1997 US 949083**
       **10.10.1997 US 948729**
       **10.10.1997 US 949084**
       **05.05.1998 US 72872**
       **05.05.1998 US 72941**
       **05.05.1998 US 72835**
       **05.05.1998 US 72834**
       **05.05.1998 US 73050**
       **05.05.1998 US 72650**

(43) Date of publication of application:
**09.08.2000 Bulletin 2000/32**

(60) Divisional application:
**02006420.0 / 1 224 917**

(73) Proprietor: **Boston Scientific Limited**
**St. Michael, Barbados (BB)**

(72) Inventors:
• **SWANSON, David K.**
 **Mountain View, CA 94040 (US)**

• **FLEISCHMAN, Sidney, D.**
 **Menlo Park, CA 94025 (US)**
• **KOBLISH, Josef, V.**
 **Sunnyvale, CA 94087 (US)**
• **THOMPSON, Russell, B.**
 **Los Altos, CA 94022 (US)**
• **WHAYNE, James, G.**
 **Saratoga, CA 95070 (US)**
• **JENKINS, Thomas R.**
 **Oakland, CA 94606 (US)**
• **SNYDER, Edward, J.**
 **San Jose, CA 95125 (US)**
• **BURNSIDE, Robert**
 **Mountain View, CA 94040 (US)**
• **YANG, Yi**
 **San Francisco, CA 94116 (US)**

(74) Representative: **Viering, Jentschura & Partner**
**Postfach 22 14 43**
**80504 München (DE)**

(56) References cited:
**EP-A- 0 530 400         WO-A-93/08755**
**WO-A-95/10236         WO-A-96/37156**
**WO-A-97/17027         WO-A-97/30644**
**DE-A- 19 503 702         DE-C- 4 425 195**
**US-A- 5 324 288**

## Description

## BACKGROUND OF THE INVENTION

### 1. Field of Invention

The present invention relates generally to structures for positioning one or more diagnostic or therapeutic elements within the body and, more particularly, to devices which are particularly well suited for treatment of cardiac conditions.

### 2. Description of the Related Art

There are many instances where diagnostic and therapeutic elements must be inserted into the body. One instance involves the treatment of cardiac conditions such as atrial fibrillation and atrial flutter which lead to an unpleasant, irregular heart beat, called arrhythmia.

Normal sinus rhythm of the heart begins with the sinoatrial node (or "SA node") generating an electrical impulse. The impulse usually propagates uniformly across the right and left atria and the atrial septum to the atrioventricular node (or "AV node"). This propagation causes the atria to contract in an organized way to transport blood from the atria to the ventricles, and to provide timed stimulation of the ventricles. The AV node regulates the propagation delay to the atrioventricular bundle (or "HIS" bundle). This coordination of the electrical activity of the heart causes atrial systole during ventricular diastole. This, in turn, improves the mechanical function of the heart. Atrial fibrillation occurs when anatomical obstacles in the heart disrupt the normally uniform propagation of electrical impulses in the atria. These anatomical obstacles (called "conduction blocks") can cause the electrical impulse to degenerate into several circular wavelets that circulate about the obstacles. These wavelets, called "reentry circuits," disrupt the normally uniform activation of the left and right atria.

Because of a loss of atrioventricular synchrony, the people who suffer from atrial fibrillation and flutter also suffer the consequences of impaired hemodynamics and loss of cardiac efficiency. They are also at greater risk of stroke and other thromboembolic complications because of loss of effective contraction and atrial stasis.

Although pharmacological treatment is available for atrial fibrillation and flutter, the treatment is far from perfect. For example, certain antiarrhythmic drugs, like quinidine and procainamide, can reduce both the incidence and the duration of atrial fibrillation episodes. Yet, these drugs often fail to maintain sinus rhythm in the patient. Cardioactive drugs, like digitalis, Beta blockers, and calcium channel blockers, can also be given to control the ventricular response. However, many people are intolerant to such drugs. Anticoagulant therapy also combats thromboembolic complications, but does not eliminate them. Unfortunately, pharmacological remedies often do not remedy the subjective symptoms associated with an irregular heartbeat. They also do not restore cardiac hemodynamics to normal and remove the risk of thromboembolism.

Many believe that the only way to really treat all three detrimental results of atrial fibrillation and flutter is to actively interrupt all of the potential pathways for atrial reentry circuits.

One surgical method of treating atrial fibrillation by interrupting pathways for reentry circuits is the so-called "maze procedure" which relies on a prescribed pattern of incisions to anatomically create a convoluted path, or maze, for electrical propagation within the left and right atria. The incisions direct the electrical impulse from the SA node along a specified route through all regions of both atria, causing uniform contraction required for normal atrial transport function. The incisions finally direct the impulse to the AV node to activate the ventricles, restoring normal atrioventricular synchrony. The incisions are also carefully placed to interrupt the conduction routes of the most common reentry circuits. The maze procedure has been found very effective in curing atrial fibrillation. However, the maze procedure is technically difficult to do. It also requires open heart surgery and is very expensive. Thus, despite its considerable clinical success, only a few maze procedures are done each year.

More recently, maze-like procedures have been developed utilizing catheters which can form lesions on the endocardium to effectively create a maze for electrical conduction in a predetermined path. Exemplary catheters are disclosed in commonly assigned U. S. Patent No. 5,582,609. Typically, the lesions are formed by ablating tissue with an electrode carried by the catheter. Electromagnetic radio frequency ("RF") energy applied by the electrode heats, and eventually kills (i.e. "ablates"), the tissue to form a lesion. During the ablation of soft tissue (i.e. tissue other than blood, bone and connective tissue), tissue coagulation occurs and it is the coagulation that kills the tissue. Thus, references to the ablation of soft tissue are necessarily references to soft tissue coagulation. "Tissue coagulation" is the process of cross-linking proteins in tissue to cause the tissue to jell. In soft tissue, it is the fluid within the tissue cell membranes that jells to kill the cells, thereby killing the tissue.

Catheters used to create lesions (the lesions being 3 to 15 cm in length) typically include a relatively long and relatively flexible body portion that has an ablation electrode on its distal end. The portion of the catheter body portion that is inserted into the patient is typically from 23 to 55 inches (58.4 to 139.7 cm) in length and there may be another 8 to 15 inches (20.3 to 38.1 cm), including a handle, outside the patient. The proximal end of the catheter body is connected to the handle which includes steering controls. The length and flexibility of the catheter body allow the catheter to be inserted into a main vein or artery (typically the femoral artery), directed into the interior of the heart, and then manipulated such that the ablation electrode contacts the tissue that is to be ablated. Fluoroscopic imaging is used to provide the physician with a visual indication of the location of the catheter.

Atrial appendages are primary potential sources of thrombus formation. The atrial appendages are especially important in the transport of blood because they have a sack-like geometry with a neck potentially more narrow than the pouch. In this case, contraction of the appendage is essential to maintain an average absolute blood velocity high enough to eliminate potential stasis regions which may lead to thrombus formation.

In the maze procedure performed through open heart surgery, the typical access points into the interior of the atria are the atrial appendages. Therefore, at the conclusion of the surgical procedure, the region occupied by the atrial appendages is eliminated by surgically removing the appendages. This mitigates subsequent problems resulting from blood stasis in the atrial appendages as well as from electrical isolation of the appendages from the rest of the atria. However, as noted above, open heart surgery is very expensive and the incision based maze procedure is difficult to perform. Although catheter-based procedures do not admit themselves to surgical removal of the appendages, catheter-based procedures and apparatus have been recently developed which reposition the atrial appendages, affix them in an altered position and/or fuse the walls of the appendages to one another to isolate the appendages, reduce stasis regions and ultimately thrombus formation. One of these procedures involves the use of a catheter having a lasso which is tightened around the appendage. RF energy is then transmitted to the appendage by way of the lasso to thermally fuse the walls of the appendage to one another, thereby isolating the appendage.

It is believed the treatment of atrial fibrillation and flutter requires the formation of long, thin lesions of different lengths and curvilinear shapes in heart tissue. Such long, curvilinear lesion patterns require the deployment within the heart of flexible ablating elements having multiple ablating regions. The formation of these lesions by ablation can provide the same therapeutic benefits that the complex incision patterns that the surgical maze procedure presently provides, but without invasive, open heart surgery.

With larger and/or longer multiple electrode elements comes the demand for more precise control of the ablating process. The delivery of ablating energy must be governed to avoid incidences of tissue damage and coagulum formation. The delivery of ablating energy must also be carefully controlled to assure the formation of uniform and continuous lesions, without hot spots and gaps forming in the ablated tissue.

The task is made more difficult because heart chambers vary in size from individual to individual. They also vary according to the condition of the patient. One common effect of heart disease is the enlargement of the heart chambers. For example, in a heart experiencing atrial fibrillation, the size of the atrium can be up to three times that of a normal atrium.

Catheter-based ablation and atrial appendage isolation have proven to be a significant advance over the conventional open heart surgery based approaches. Nevertheless, the inventors herein have determined that further improvements are possible.

For example, and with respect to ablation procedures in particular, the inventors herein have determined that it can be quite difficult to accurately position an ablation electrode on the endocardium surface by manipulating the distal end of a relatively long catheter body from a remote handle. This is especially true with respect to left atrial sites. The present inventors have also determined that fluoroscopy is a somewhat inaccurate method of visualizing the ablation electrodes during positioning and when determining whether the electrodes are in proper contact with tissue.

Additionally, a primary goal of any ablation procedure is to create contiguous lesions (often long, curvilinear lesions) without over-heating tissue and causing coagulum and charring. Tissue ablation occurs at 50°C, while over-heating occurs at 100°C. The present inventors have further determined that it can be difficult to produce tissue contact that will accomplish this result with an electrode mounted on the distal end of a relatively long catheter. This is especially true in those procedures where an electrode on the distal tip of the catheter is dragged along the tissue. Such dragging also makes accurate placement of the electrode very difficult. Other shortcomings identified by the present inventors concern the convective cooling effects of the blood pool on the electrodes. For example, the system power requirements must be high enough to compensate for the heat losses due to convective cooling.

One proposed method of solving the overheating problems associated with conventional ablation catheters is the so-called "cooled tip" approach. Here, the tissue surface is cooled with a saline solution. Although the saline is somewhat useful in keeping the surface temperature below the over-heating temperature, the sub-surface tissue temperature can still rise well above 100°C. Such temperatures will cause gas within the sub-surface tissue to expand. Ultimately, the tissue will tear or pop, which will result in perforations of the epicardial surface and/or the dislodging of chunks of tissue that can cause strokes.

Turning to atrial appendage isolation, the present inventors have determined that catheter-based procedures suffer from many of the same disadvantages discussed above, such as those concerning positioning and visualization. Additionally, the inventors herein have determined that the lasso can bunch up the tissue when the lasso is tightened and that tissue fusion would be improved if this bunching could be avoided.

With respect to energy control, conventional ablation devices include controls that are located either on the RF energy source, or on a foot pedal. The inventors herein have determined that such arrangements are inconvenient and can make it difficult to control power during a surgical procedure.

Turning to surgical procedures in general, one problem associated with many surgical procedures is excessive bleeding. For example, a high level of bleeding is often associated with the removal of liver lobes and certain cancerous tumors. The inventors herein have determined that present surgical methods could be improved in the area of blood loss.

WO-A-9 510 236 discloses a coagulation probe comprising a handle and coagulation electrodes supported on an electrode support assembly having an electromagnetic energy emitting region creating a single continuous lesion when caused to emit energy.

EP-A-0 530 400 discloses a surgical instrument that includes a tubular sleeve supported on a tubular shaft, wherein the tubular sleeve and the tubular shaft are both plastically deformable.

## SUMMARY OF THE INVENTION

The general object of the present invention is to provide an apparatus for positioning an operative element (such as an ablation electrode) within the body which avoids, for practical purposes, the aforementioned problems. This is achieved by the features according to claim 1. Preferred embodiments of the invention are disclosed in the subclaims, respectively. In particular, one object of the invention is to provide tissue ablation systems providing beneficial therapeutic results without requiring highly invasive surgical procedures. Another objective of the invention is to provide systems that simplify the creation of complex lesions patterns in soft tissue, such as myocardial tissue in the heart.

In order to accomplish the above-described and other objectives, a surgical device in accordance with one embodiment of the present invention includes a relatively short shaft, a bendable spline assembly associated with the distal end of the shaft and having a predetermined configuration, the spline assembly being adapted to collapse in response to external forces and expand when the forces are removed, and an operative element associated with the bendable spline. Optionally, a substantially tubular member may be positioned around the shaft. Movement of the substantially tubular member over the spline assembly will cause the spline assembly to collapse, while the spline assembly will expand to the predetermined configuration in response to a retraction of the substantially tubular member.

In order to accomplish above-described and other objectives, a soft tissue coagulation probe in accordance with one embodiment of the invention includes a relatively short shaft defining a distal end and a proximal end, a handle associated with the proximal end of the shaft, and at least one soft tissue coagulation electrode associated with the shaft and located in spaced relation to the handle.

In order to accomplish above-described and other objectives, a surgical device in accordance with another embodiment of this invention includes a relatively stiff shaft, a handle associated with the proximal end of the shaft, and a distal tip assembly associated with the distal end of the shaft, the distal tip assembly including a distal member, which is flexible and/or malleable, and an operative element carried by the distal member.

In order to accomplish this and other objectives, a surgical device in accordance with another embodiment of this invention includes a shaft, a relatively stiff tubular member positioned around a predetermined portion of the shaft and movable relative thereto, a distal tip assembly associated with the distal end of the shaft and including a flexible distal member and an operative element carried by the distal member, and a pivot assembly associated with the distal end of the tubular member and a distal portion of the tip assembly.

There are many advantages associated with the invention. For example, the above-described embodiments of this invention may be used to treat atrial fibrillation wherein access to the heart is obtained by way of a thoracostomy. Here, the operative element is an ablation electrode. The invention may also be used to treat atrial fibrillation during mitral valve surgery wherein access to the heart is obtained through a thoracostomy, thoracotomy or median sternotomy.

The relatively short shaft and manner of insertion allows the ablation electrode to be easily inserted into the atrium and visually guided to the desired location. Thus, the ablation electrodes in the present device do not have to be guided by manipulating the relatively long shaft of an endovascular catheter. This makes the positioning of the electrodes within the heart easier and more accurate. Endocardial visualization is also improved because surgical procedures employing the present device allow the endocardium to be viewed directly with the naked eye, a fiberoptic camera or other imaging modalities. This eliminates the need for fluoroscopic images and reduces the amount of radiation required, as compared to catheter-based procedures. Moreover, the shaft in the present device can be relatively stiff, as compared to a catheter shaft, because the present shaft does not have to travel through the tortuous vascular path to the heart. Along with the relatively short length of the present shaft, the additional stiffness enhances torque transmission and provides superior and more reliable electrode-endocardium contact force.

Surgical devices in accordance with this invention may also be used during procedures, such as valve replacement where the patient is on cardiopulmonary bypass, to create tissue lesions. During bypass, the electrodes elements will not be in contact with the blood pool and, accordingly, will not be affected by the convective cooling.

Patients can only be on bypass for a period of approximately four hours. Long bypass times are associated with increased morbidity and mortality. Thus, all procedures performed during bypass must be rapidly completed. Surgical devices in accordance with the present invention may include a series of temperature controlled electrodes that allow a long lesion to be created in rapid fashion, i.e. in approximately 30 to 120 seconds. The ability of the present surgical devices and techniques to create lesions rapidly allows procedures to be performed during bypass that, heretofore, could not due to the time constraints. For example, a conventional surgical maze procedure takes approximately 12 hours to complete (note that a portion of the procedure is performed while the patient is not on bypass), while such a procedure may be completed in approximately 5 to 15 minutes with the present devices.

In accordance with another advantageous aspect of this invention, the shaft and/or sheath (if present) may be formed from a malleable material that a physician can bend into a desired configuration and remain in that configuration when released. Although malleable, the stiffness of such material must be at least such that the shaft and/or sheath (if present) will not bend under the forces applied thereto during a surgical procedure. Therefore, the shaft has a bending modulus of between approximately 3 lb.-in.$^2$ (86 N-cm$^2$) and approximately 50 lb.-in.$^2$ (1435 N-cm$^2$), wherein the bending modulus is the product of the modulus of elasticity and of the moment of inertia of the shaft. Alternatively, or in addition, the distal end of the device may also be malleable, thereby allowing the physician to bend the distal end of the device into a shape corresponding to the bodily structure to be acted upon. This is particularly important in endocardial applications because the endocardial surface is typically non-uniform with ridges and trabeculae residing in the right and left atria. There are also dramatic differences between endocardial surface morphology from patient to patient and from lesion location to lesion location. To create contiguous lesions with a surgical approach, the device must either distend the atria to flatten out the nonuniformities, or the probe must be configured to conform to the atrial surface. There are, however, some regions where the atria cannot be distended to a flat state because of trabeculae, orifices, and ridges. A surgeon can observe the atrial surface and bend the present malleable device so as to conform thereto. The distal end may, instead, be spring-like or even rigid if the application so requires.

In order to accomplish the above-identified and other objectives, a surgical device in accordance with one embodiment of the present invention includes a handle having at least one movable handle member, first and second support members operably connected to the handle, at least one of the support members being movable with respect to the other support member in response to movement of the at least one movable handle member, and at least one ablation electrode associated with the first support member.

There are many advantages associated with this invention. By way of example, this invention is especially useful when isolating an atrial appendage. Access to the atrium may be obtained by, for example, a thoracostomy and the appendage may be captured between the support members. RF energy is then applied to the captured portion of the appendage to thermally fuse the walls of the appendage to one another. This provides better heating and fusing than the lasso catheter-based approach because the tissue is not bunched up when captured between the support members, as it is when the lasso is tightened. Additionally, the disadvantages associated with the use of catheters in general are also avoided.

A surgical clamp in accordance with one embodiment of the present invention includes first and second clamp members, and at least one electrode associated with at least one of the clamp members. The clamp may be used to isolate an atrial appendage in a manner similar to that described in the preceding paragraph with the same advantageous results. Thereafter, the clamp may be either removed or left in place.

A surgical device in accordance with one embodiment of the present invention includes an energy source, at least one energy transmission device, and a handle including an energy control device coupled to the energy source and to the at least one energy transmission device. The energy control device is adapted to selectively control the transmission of energy from the energy source to the at least one energy transmission device. Because the energy control device is located on the handle, which is necessarily grasped by the physician during surgical procedures, the present surgical device provides more convenient energy control than that found in conventional devices.

Alternatively, and in accordance with one embodiment of the present invention, energy control may be accomplished through the use of a remote energy control device that is connected to power unit, but located in close proximity to the patient or otherwise within the sterile zone of an operating room. Such an arrangement also provides more convenient energy control than that found in conventional devices.

Additionally, whether the power control interface is located on the handle of a surgical probe or on a remote control device, the power control aspect of the overall electrophysiological system can be more conveniently brought into the sterile zone because both the present surgical probe and remote control device are both readily sterilizable. Conventional power control interfaces, on the other hand, are part of a power control unit that is not readily sterilizable.

To further improve tissue contact, a pressure application probe in accordance with one embodiment of the present invention may be used in conjunction with a probe having an energy transmission device on a support member. The pressure application probe includes an elongate main body portion and an engagement device adapted to releasably engage the support member. The pressure application probe can be used by the physician to insure that sufficient tissue contact is realized prior to energy transmission.

A coupling device in accordance with another embodiment of the present invention can also be used in conjunction with a probe having an energy transmission device on a support member. One embodiment of the coupling device includes a base member adapted to be removably secured to a first portion of the probe's flexible support member and an engagement device connected to the base member and adapted to be removably secured to a second portion of the flexible support member. The coupling device enables a physician to form a distal loop in the support member when desired, thereby increasing the flexibility of the probe.

In order to reduce the blood loss associated certain surgical procedures, a surgical procedure employing the present invention includes the steps of coagulating soft tissue and then forming an incision is the coagulated tissue. If the incision is no deeper than the coagulation, the incision will not result in significant bleeding. This process can be repeated until an incision of the desired depth is achieved.

The above described and many other features and attendant advantages of the present invention will become apparent as the invention becomes better understood by reference to the following detailed description when considered in conjunction with the accompanying drawings.

## BRIEF DESCRIPTION OF THE DRAWINGS

Detailed description of preferred embodiments of the invention will be made with reference to the accompanying drawings.

FIGURE 1 is a side, partial section view of a surgical device for positioning an operative element within a patient in accordance with a preferred embodiment of the present invention.

FIGURE 2 is an end view of the surgical device shown in FIGURE 1.

FIGURE 3a is a side view of a surgical device for positioning an operative element within a patient in accordance with another preferred embodiment of the present invention.

FIGURE 3b is a partial side view of a portion of the surgical device shown in FIGURE 3a.

FIGURE 4 is a side, partial section view of a portion of the surgical device shown in FIGURE 3a.

FIGURE 5 is a side view of a surgical device for positioning an operative element within a patient in accordance with still another preferred embodiment of the present invention.

FIGURE 6a is a partial side, cutaway view of a surgical device for positioning an operative element within a patient in accordance with yet another preferred embodiment of the present invention.

FIGURE 6b is a section view taken along line 6b-6b in FIGURE 6a.

FIGURE 7 is a section view showing an operative element coated with regenerated cellulose.

FIGURE 8a is a section view showing a partially masked operative element.

FIGURE 8b is a section view showing an alternative operative element configuration.

FIGURES 9a-9c are front views of a spline assembly in accordance with an embodiment of the present invention.

FIGURE 9d is a side view of the spline assembly shown in FIGURES 9a-9c.

FIGURE 9e is a section view taken along line 9e-9e in FIGURE 9a.

FIGURE 9f is a partial front, partial section view of a surgical device for positioning an operative element within a patient in accordance with yet another preferred embodiment of the present invention.

FIGURE 10a is a side view of a surgical device for positioning an operative element within a patient in accordance with a preferred embodiment of the present invention.

FIGURE 10b is a side, partial section view of an alternate tip that may be used in conjunction with the device shown in FIGURE 10a.

FIGURE 10c is a side, section view of another alternate tip that may be used in conjunction with the device shown in FIGURE 10a.

FIGURE 10d is a perspective view of a probe handle in accordance with one embodiment of the present invention.

FIGURE 10e is a perspective view of a probe handle in accordance with another embodiment of the present invention.

FIGURE 10f is an exploded perspective view of a probe in accordance with one embodiment of the present invention.

FIGURE 10g is an enlarged view of a portion of the probe shown in FIGURE 10f.

FIGURE 10h is a plan view of an electrophysiology system in accordance with one embodiment of the present invention.

FIGURE 10i is an enlarged view of the remote power control unit shown in FIGURE 10h.

FIGURE 11a is a section view of the distal portion of the device shown in FIGURE 10a taken along line 11a-11a in FIGURE 10a.

FIGURE 11b a section view of an alternate distal portion for the device shown in FIGURE 10a.

FIGURE 11c is a side, partial section view of another alternative distal portion for the device shown in FIGURE 10a.

FIGURE 12 is a section view taken along line 12-12 in FIGURE 10a.

FIGURE 13 is a side view of a surgical device for positioning an operative element within a patient in accordance with another preferred embodiment of the present invention.

FIGURE 14 is a side view of a surgical device for positioning an operative element within a patient in accordance with yet another preferred embodiment of the present invention.

FIGURE 15 is a perspective view of a portion of the device shown in FIGURE 14.

FIGURE 16 is a side view of a surgical device for positioning an operative element within a patient in accordance with still another preferred embodiment of the present invention.

FIGURE 17 is a side view of a clamp in accordance with a preferred embodiment of the present invention.

FIGURE 18 is a section view taken along line 18-18 in FIGURE 17.

FIGURE 19 is a top view of the clamp illustrated in FIGURE 17.

FIGURE 20 is a side view of a surgical device for positioning an operative element within a patient and applying a clamping force to a bodily structure in accordance with a preferred embodiment of the present invention.

FIGURE 21 is a side view of a surgical device for positioning an operative element within a patient and applying a clamping force to a bodily structure in accordance with another preferred embodiment of the present invention.

FIGURE 22 is a side view of a surgical device for positioning an operative element within a patient and applying a clamping force to a bodily structure in accordance with still another preferred embodiment of the present invention.

FIGURE 23 is a top view of the operative element supporting member of the surgical device shown in FIGURE 22.

FIGURE 24a is a top view of another operative element supporting member.

FIGURE 24b is a top view of still another operative element supporting member.

FIGURE 25 is a side view of a surgical device for positioning an operative element within a patient and applying a clamping force to a bodily structure in accordance with yet another preferred embodiment of the present invention.

FIGURE 26 is a side, partial section view of an exemplary procedure involving the surgical device shown in FIGURE 20.

FIGURE 27 is a side, partial section view of an exemplary procedure involving a surgical device having an alternate support member configuration.

FIGURES 28 and 29 are schematic views of a system for controlling the application of ablating energy to multiple electrodes using multiple temperature sensing inputs.

FIGURE 30 is a schematic flow chart showing an implementation of the temperature feedback controller shown in FIGURES 28 and 29, using individual amplitude control with collective duty cycle control.

FIGURE 31 is a schematic view of a neural network predictor, which receives as input the temperatures sensed by multiple sensing elements at a given electrode region and outputs a predicted temperature of the hottest tissue region.

FIGURE 32 is a fragmentary side view showing the use of a grabbing catheter in conjunction with a lasso catheter for maintaining the walls of the inverted appendage together.

FIGURE 33 is a fragmentary view of the combination shown in FIGURE 32 illustrating further steps of tying an appendage in an inverted orientation.

FIGURE 34 is a perspective view of a pressure application probe in accordance with a preferred embodiment of the present invention secured to an operative element supporting probe.

FIGURE 35 is an enlarged perspective view of the pressure application probe shown in FIGURE 34.

FIGURE 36 is a partial perspective view of a pressure application probe in accordance with another preferred embodiment of the present invention.

FIGURE 37 is a perspective view of a coupling device in accordance with a preferred embodiment of the present invention.

FIGURE 38 is a perspective view showing a pressure application probe and the coupling device shown in FIGURE 37 being used in combination with the surgical device shown in FIGURE 10a.

FIGURE 39 is a perspective view showing the coupling device shown in FIGURE 37 being used in combination with the surgical device shown in FIGURE 10a.

FIGURE 40 is a perspective view of a coupling device in accordance with another preferred embodiment of the present invention.

## DETAILED DESCRIPTION OF THE PREFERRED EMBODIMENTS

The following is a detailed description of the best presently known modes of carrying out the invention. This description is not to be taken in a limiting sense, but is made merely for the purpose of illustrating the general principles of the invention.

The detailed description of the preferred embodiments is organized as follows:

    I. Probe-Type Apparatus
    II. Operative Elements
    III. Epicardial Applications of Probe-Type Apparatus
    IV. Endocardial Applications of Probe-Type Apparatus

V. Other Surgical Applications

VI. Apparatus that Apply a Clamping Force

VII. Applications of Apparatus that Apply a Clamping Force

VIII. Power Control

The section titles and overall organization of the present detailed description are for the purpose of convenience only and are not intended to limit the present invention.

This specification discloses a number of electrode structures, mainly in the context of cardiac ablation, because the structures are well suited for use with myocardial tissue. Nevertheless, it should be appreciated that the structures are applicable for use in therapies involving other types of soft tissue. For example, various aspects of the present invention have applications in procedures concerning other regions of the body such as the prostate, liver, brain, gall bladder, uterus and other solid organs.

**I. Probe-Type Apparatus**

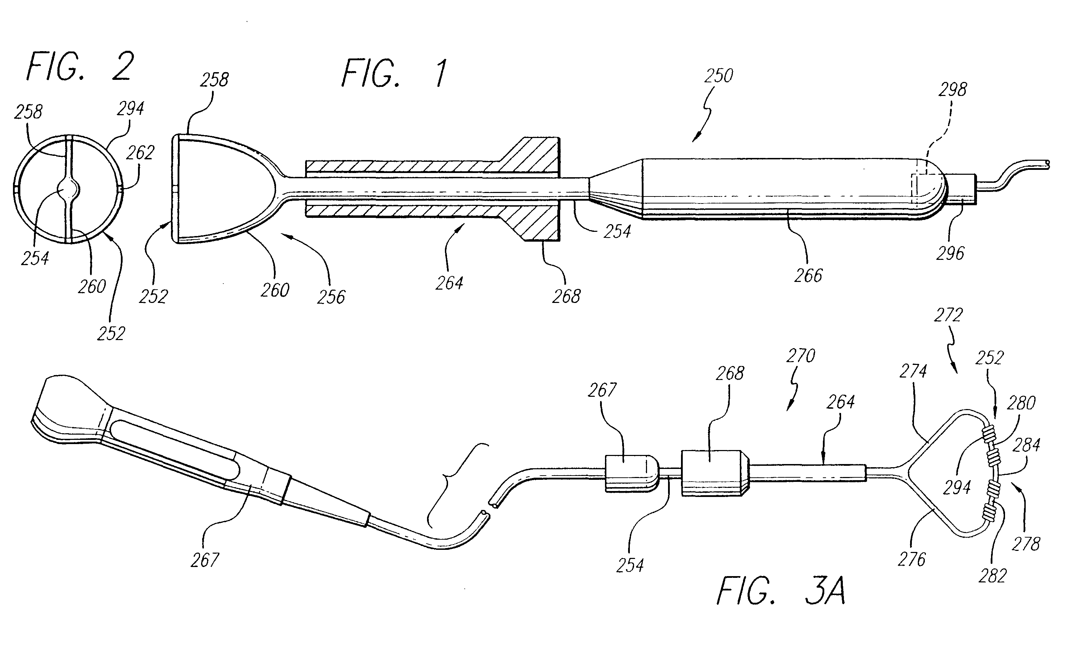

As illustrated for example in FIGURES 1 and 2, a surgical device (or "probe") 250 for positioning an operative element 252 within a patient includes a relatively short shaft 254 and a bendable spline assembly 256, associated with the distal end of the shaft, for supporting the operative element. Here, the operative element 252 is in the form of a plurality of electrode elements 294, as discussed in greater detail in Section II below. Preferably, the relatively short shaft may be between approximately 4 and 18 inches (10.2 and 45.7 cm) in length, and is preferably 8 inches (20.3 cm) in length, while the outer diameter of the shaft is preferably between approximately 6 and 24 French (2 and 8 mm). The spline assembly 256 has a predetermined use configuration. In the exemplary embodiment shown in FIGURES 1 and 2, the spline assembly includes a pair of spline legs 258 and 260 and an annular member 262 which supports the operative element 252. The surgical device also includes a tubular member 264 (a cylindrically shaped sheath in the exemplary embodiment) which covers a portion of the shaft 254 and is also slidable relative thereto. The spline assembly 256 is adapted to collapse (the insertion configuration) in response to movement of the substantially tubular member 264 in the distal direction and to expand to the predetermined use configuration when the substantially tubular member is moved in the proximal direction. A handle 266 may be provided on the proximal end of the shaft 254. The tubular member 264 preferably includes a raised gripping surface 268.

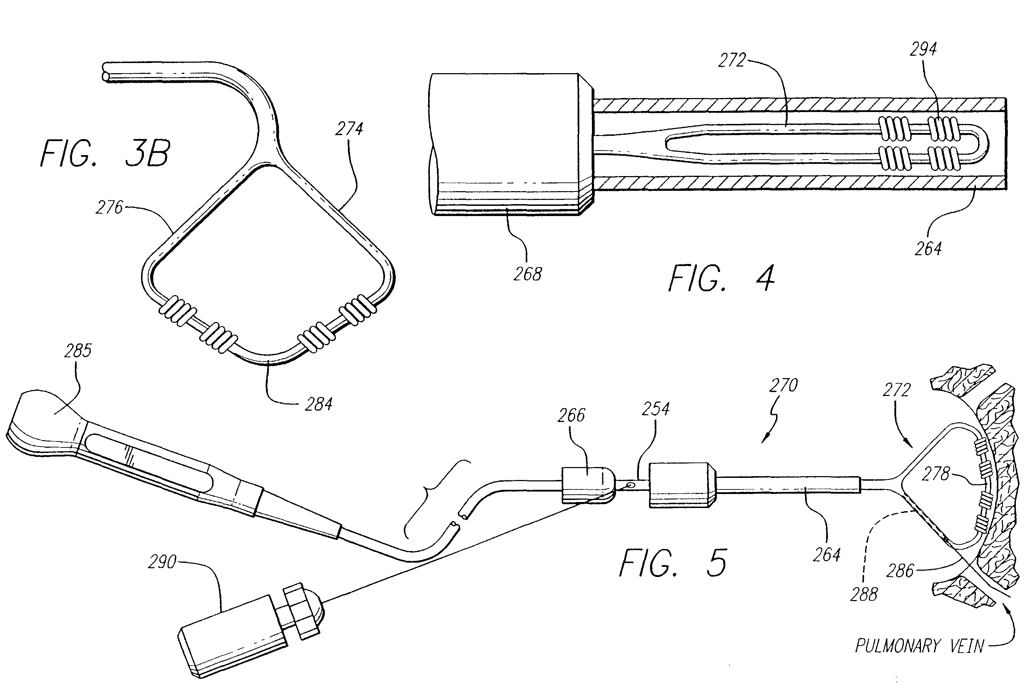

Another exemplary surgical device (or "probe") for positioning an operative element within a patient, which is generally represented by reference numeral 270, is illustrated in FIGURES 3a-4. Here, the surgical device includes a substantially triangularly shaped spline assembly 272 that consists of first and second side legs 274 and 276 and a distal leg 278. The distal leg 278, which is preferably non-linear from end to end and approximately 10 to 12 cm in length, includes first and second linear portions 280 and 282 and a bent portion 284 located mid-way between the ends. This spline configuration provides a spring force against the selected bodily surface during use (such as the atrium wall in a cardiac procedure) and the bend in the distal leg 278 optimizes the contact between the operative element 252 and the selected surface. The spline assembly 272 will collapse in the manner shown in FIGURE 4 when the tubular member 264 is advanced thereover and will return to the orientation shown in FIGURE 3a when the tubular member is retracted. The surgical device 270 also includes a second handle 267.

During use of the exemplary surgical device shown in FIGURES 1-4, the handle 266 (FIGURE 1) or 267 (FIGURE 3a) is grasped by the physician and force is applied through the shaft 254 and side legs 258 and 260 (FIGURE 1) or 274 and 276 (FIGURE 3a) to the operative element supporting annular member 262 (FIGURE 1) or distal leg 278 (FIGURE 3a). Thus, the shaft and side legs (including the area where the side legs meet) should be sufficiently strong to prevent collapse when the force is applied. The fact that the present devices are not passed through a tortured vascular path to the site of interest allows the shaft and spline legs to be stiffer than a conventional catheter shaft. This aspect of the invention is discussed in greater detail below. Alternatively, the shaft 254 and side legs 274 and 276 in the embodiment shown in FIGURES 3a and 4 may be configured such that they collapse and form a semicircle with the distal leg 278 when force is applied to the shaft (note FIGURE 3b). Here, the operative element should be appropriately masked in one of the manners described below to limit contact of the operative element to the intended bodily structure.

As shown by way of example in FIGURE 5, a guidewire 286 may be used to direct and/or anchor the distal leg 278 of the exemplary spline assembly 272 in an anatomical anchor site (such as one of the pulmonary veins shown in FIGURE 5). The guidewire 286 passes through a lumen in the shaft 254. The distal end of the guidewire 286 passes through a lumen 288 formed in one of the spline assembly side legs 274 and 276, while the proximal end is secured to a handle 290. Alternatively, two guide wires (one passing through each of the side legs) may be used to anchor the spline assembly 272 in two anatomical anchor sites. Both wires would extend to the same handle.

The exemplary embodiments illustrated in FIGURES 1-5 may also be provided without the tubular member 264. Such devices are especially useful in surgical procedures associated with a thoracotomy or a median stemotomy, where the spline assemblies can be easily collapsed and advanced to the desired location, or advanced into the desired location without being collapsed. Here, the spline assemblies can be malleable, if desired, as opposed to simply being bendable.

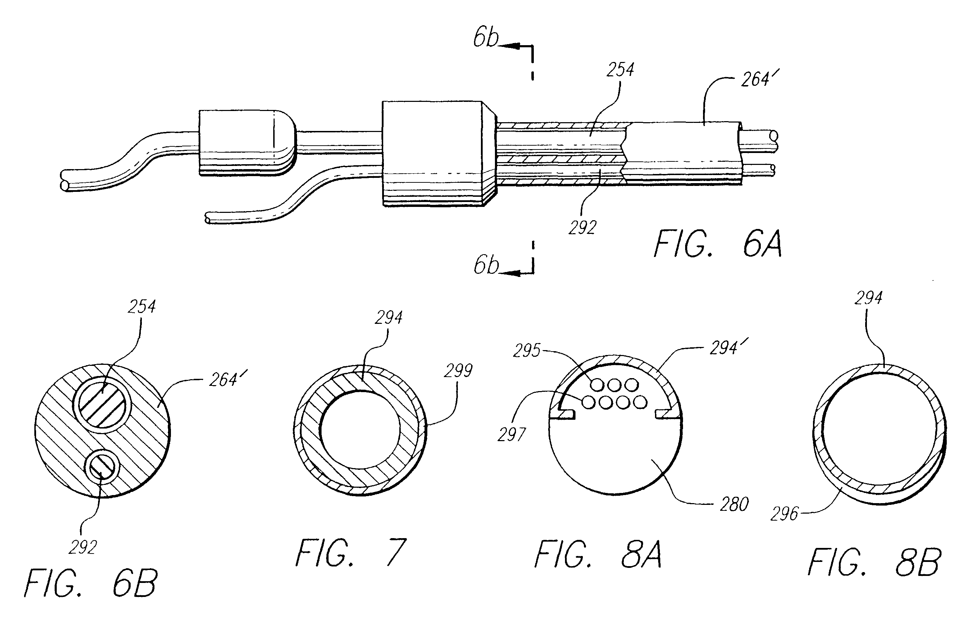

Turning to FIGURES 6a and 6b, an endoscope 292 may be passed through one lumen in a tubular member 264' that has a pair of lumens. Alternatively, the shaft 254 and endoscope 292 can pass through a common lumen.

The spline assemblies illustrated in FIGURES 1-5 are preferably made from resilient, inert wire, like nickel titanium (commercially available as Nitinol material) or 17-7 stainless steel. However, resilient injection molded inert plastic can also be used. The wire or molded plastic is covered by suitable biocompatible thermoplastic or elastomeric material such as PEBAX® or Pellethane®. Preferably, the various portions of the spline assemblies comprises a thin, rectilinear strips of resilient metal or plastic material. Still, other cross-sectional and longitudinal configurations can be used. For example, the spline legs can decrease in cross-sectional area in a distal direction, by varying, e.g., thickness or width or diameter (if round), to provide variable stiffness along its length. Variable stiffness can also be imparted by composition changes in materials or by different material processing techniques. Referring more specifically to the embodiments illustrated in FIGURES 3a-5, the distal leg 278 may be configured such that the leg is flat at the distal end, but becomes more semicircular in cross-section as the leg becomes more proximal in order to taper the stiffness profile and prevent lateral movement of the spline assembly. The curvature of the spline legs may also be varied and the lateral ends of the distal leg may be reinforced in order to provide more lateral stability.

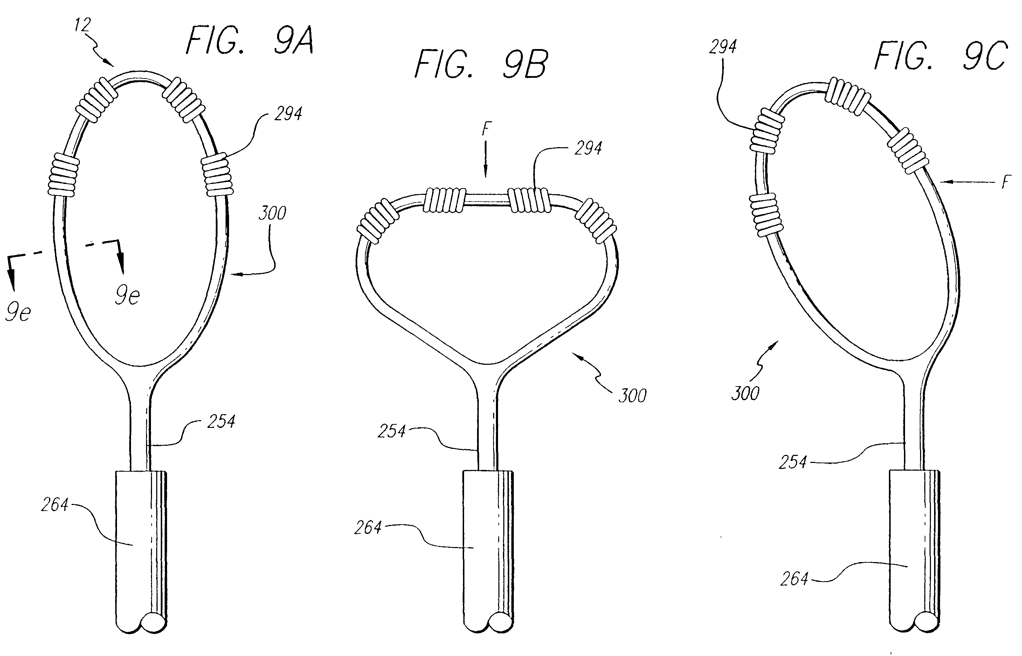

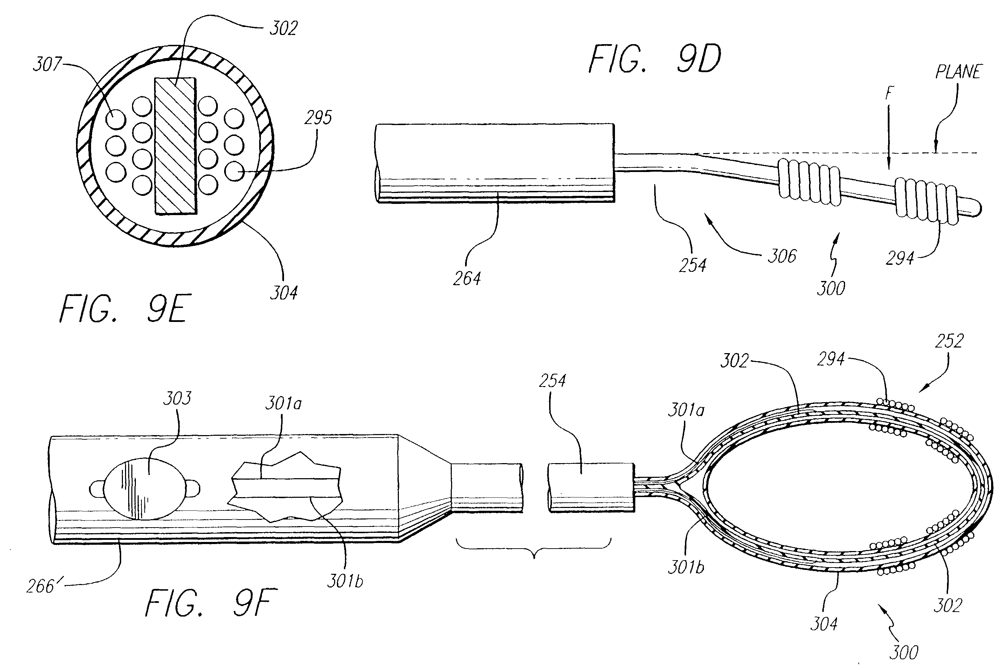

As shown by way of example in FIGURES 9a-9e, the spline assembly of the probe shown in FIGURES 3a and 4 may be replaced by a curved spline assembly 300. Here, the spline assembly includes a flat, inert wire 302 (preferably formed from Nitinol) that acts as a spring and an outer portion 304 (preferably formed from PEBAX® or Pellethane®). Viewed in cross-section, the flat wire 302 has a long side and a short side. The short sides lie in planes that are parallel to the plane shown in FIGURE 9d. As such, the spline assembly 300 will deflect in the manner shown in FIGURES 9b and 9c when "in plane" forces F are applied to the spline assembly. Conversely, the assembly will resist bending when "out of plane" forces are applied in the manner shown in FIGURE 9d. As such, it may be used to form an arcuate lesion during, for example, a procedure where a lesion is formed around the pulmonary vein.

It should be noted here that the wire 302 does not have to be rectangular in cross-section as shown. Other cross-sectional shapes where the length is greater than the width can also be used. The wire 302 can also be made from a malleable material such as partially or fully annealed stainless steel instead of the spring-like material discussed above. The malleable embodiments will enable the operator to form fit the ablation element support structure to irregular anatomical structures.

As shown in FIGURE 9f, exemplary spline assembly 300' includes first and second steering wires 301a and 301b that are secured to the spring-like flat wire 302 by, for example, welding, mechanical crimping or adhesive bonding. The proximal ends of the steering wires 301a and 301b are operably connected to a knob 303 on a handle 266' by way of a cam (not shown). The handle 266' is substantially similar to the handle 266 shown in FIGURE 1, but for the knob 303, cam and provisions for the steering wires 301a and 301 b. Rotation of the knob 303 will cause the spline assembly to move side to side in, for example, the manner illustrated in FIGURE 9c. Thus, in addition to simply moving the handle, the physician will be able to move the operative element 252 within the patient by rotating the knob 303. Such movement is useful when the physician is attempting to precisely locate the operative element within the patient and/or control the contact force between the operative element and the tissue surface. This is especially true when the handle and or shaft 254 cannot be moved, due to anatomical or surgical constraints.

In the exemplary embodiment, the steering wires 301 a and 301b are both secured at about the midpoint of the flat wire loop. Other configurations are possible depending on the configuration of the loop that is desired after the knob 303 is rotated. For example, one wire could be secured closer to the top of the loop than the other. The shape of the cam may also be varied. More detailed discussions of the use of steering wires, albeit in conventional catheter settings, can be found in commonly assigned U.S. Patent Nos. 5,195,968, 5,257,451, and 5,582,609.

The shaft 254 is preferably relatively stiff. As used herein the phrase "relatively stiff' means that the shaft (or other structural element) is either rigid, malleable, or somewhat flexible. A rigid shaft cannot be bent. A malleable shaft is a shaft that can be readily bent by the physician to a desired shape, without springing back when released, so that it will remain in that shape during the surgical procedure. Thus, the stiffness of a malleable shaft must be low enough to allow the shaft to be bent, but high enough to resist bending when the forces associated with a surgical procedure are applied to the shaft. A somewhat flexible shaft will bend and spring back when released. However, the force required to bend the shaft must be substantial. Rigid and somewhat flexible shafts are preferably formed from stainless steel, while malleable shafts are formed from annealed stainless steel.

One method of quantifying the flexibility of a shaft, be it shafts in accordance with the present invention or the shafts of conventional catheters, is to look at the deflection of the shaft when one end is fixed in cantilever fashion and a force normal to the longitudinal axis of the shaft is applied somewhere between the ends. Such deflection ($\sigma$) is expressed as follows:

$$\sigma = WX^2(3L-X)/6EI$$

where:

W is the force applied normal to the longitudinal axis of the shaft,
L is the length of the shaft,
X is the distance between the fixed end of the shaft and the applied force,
E is the modulous of elasticity, and
I is the moment of inertia of the shaft.

When the force is applied to the free end of the shaft, deflection can be expressed as follows:

$$\sigma = WL^3/3EI$$

Assuming that W and L are equal when comparing different shafts, the respective E and I values will determine how much the shafts will bend. In other words, the stiffness of a shaft is a function of the product of E and I. This product is referred to herein as the "bending modulus." E is a property of the material that forms the shaft, while I is a function of shaft geometry, wall thickness, etc. Therefore, a shaft formed from relatively soft material can have the same bending modulus as a shaft formed from relatively hard material, if the moment of inertia of the softer shaft is sufficiently greater than that of the harder shaft.

For example, a relatively stiff 2 inch (5.1 cm) shaft (either malleable or somewhat flexible) would have a bending modulus of at least approximately 1 lb.-in.$^2$ (28 N-cm$^2$) Preferably, a relatively stiff 2 inch (5.1 cm) shaft will have a bending modulus of between approximately 3 lb.-in.$^2$ (86 N-cm$^2$) and approximately 50 lb.-in.$^2$ (1435 N-cm$^2$). By comparison, 2 inch (5.1 cm) piece of a conventional catheter shaft, which must be flexible enough to travel through veins, typically has bending modulus between approximately 0.1 lb.-in.$^2$ (2.8 N-cm$^2$) and approximately 0.3 lb.-in.$^2$ (8.6 N-cm$^2$). It should be noted that the bending modulus ranges discussed here are primarily associated with initial deflection. In other words, the bending modulus ranges are based on the amount of force, applied at and normal to the free end of the longitudinal axis of the cantilevered shaft, that is needed to produce 1 inch (2.5 cm) of deflection from an at rest (or no deflection) position.

As noted above, the deflection of a shaft depends on the composition of the shaft as well as its moment of inertia. The shaft could be made of elastic material, plastic material, elasto-plastic material or a combination thereof. By designing the shaft 254 to be relatively stiff (and preferably malleable), the surgical tool is better adapted to the constraints encountered during the surgical procedure. The force required to bend a relatively stiff 2 inch (5.1 cm) long shaft should be in the range of approximately 1.5 lbs. (6.7 N) to approximately 12 lbs. (53.4 N). By comparison, the force required to bend a 2 inch (5.1 cm) piece of conventional catheter shaft should be between approximately 0.2 lb. (0.9 N) to 0.25 lb. (1.1 N). Again, such force values concern the amount of force, applied at and normal to the free end of the longitudinal axis of the cantilevered shaft, that is needed to produce 1 inch (2.5 cm) of deflection from an at rest (or no deflection) position.

Ductile materials are preferable in many applications because such materials can deform plastically before failure due to fracturing. Materials are classified as either ductile or brittle, based upon the percentage of elongation when the fracture occurs. A material with more than 5 percent elongation prior to fracture is generally considered ductile, while a material with less than 5 percent elongation prior to fracture is generally considered brittle. Material ductility can be based on a comparison of the cross sectional area at fracture relative to the original cross area. This characteristic is not dependent on the elastic properties of the material.

Alternatively, the shaft could be a mechanical component similar to shielded (metal spiral wind jacket) conduit or flexible Loc-Line®, which is a linear set of interlocking ball and socket linkages that can have a center lumen. These would be hinge-like segmented sections linearly assembled to make the shaft.

The exemplary tubular member 264 illustrated in FIGURES 1-6b is preferably in the form of a relatively thin cylindrical sheath (e.g., with a wall thickness of about 0.005 inch (1.2 mm)) and has an outer diameter which is preferably less than 0.180 inch (46 mm). The sheath material is preferably also lubricious, to reduce friction during movement of the sheath relative to the shaft 254 and spline assemblies 256 and 272. For example, materials made from polytetrafluoroethylene (PTFE) can be used for the sheath. The distal end of the sheath should be relatively flexible to prevent injury. If necessary, additional stiffness can be imparted to the remaining portion of the sheath by lining the sheath with a braided material coated with PEBAX® material (comprising polyethel block amide related to nylon). Other compositions made from PTFE braided with a stiff outer layer and other lubricious materials can be used.

Alternatively, the tubular member 264 may be relatively stiff and formed from the materials described above with respect to the shaft 254.

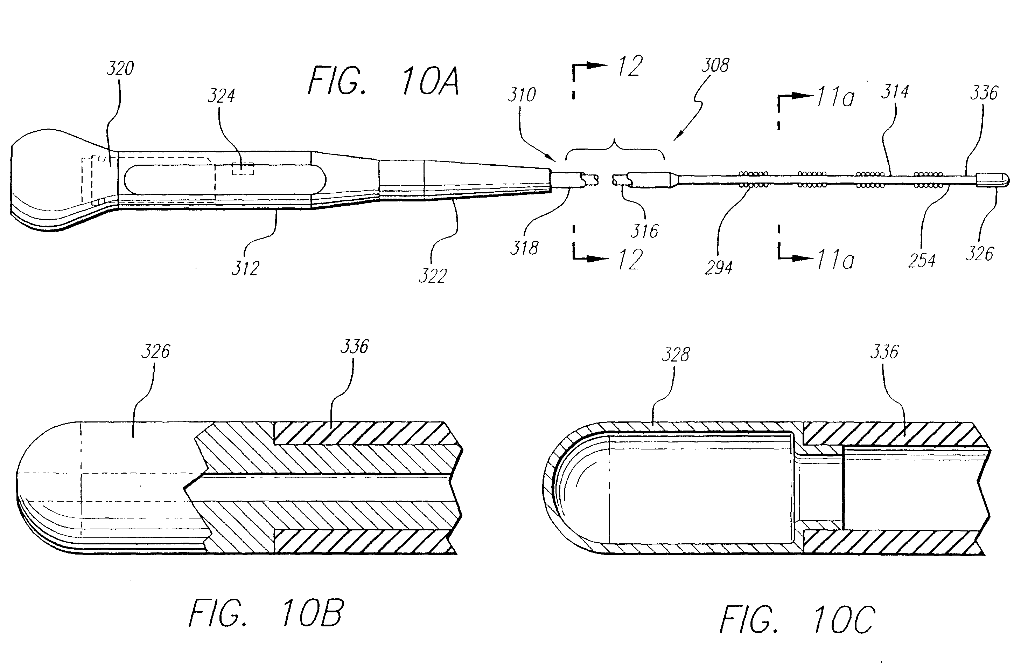

As shown by way of example in FIGURE 10a, a surgical probe 308 in accordance with another embodiment of this invention includes a relatively stiff shaft 310, a handle 312 and a distal section 314. The shaft 310 consists of a hypo-tube 316, which is either rigid or relatively stiff, and an outer polymer tubing 318 over the hypo-tube. A relatively stiff tube, either malleable or somewhat flexible, will preferably have a bending modulus of between approximately 3 lb.-in.$^2$ (86 N-cm$^2$) and approximately 50 lb.-in.$^2$ (1435 N-cm$^2$). The handle 312 is similar to the handle 266 discussed above in that it includes a PC board 320 for connecting the operative elements on the distal portion of the probe to a power source. The handle 312 preferably consists of two molded handle halves and is also provided with strain relief element 322. An operative element 254 (here, in the form of a plurality of electrode elements 294) is provided on the distal section 314. This embodiment is particularly useful because it can be easily inserted into the patient through an introducing port such as a trocar.

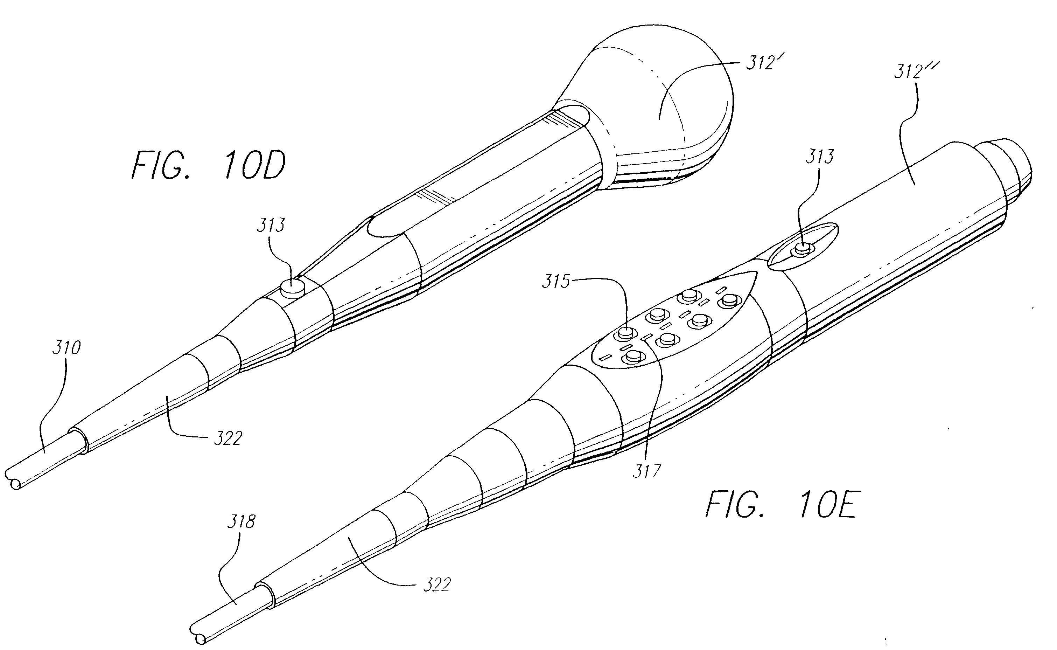

The handle 312 shown in FIGURE 10a is intended to be used in a conventional power supply configuration, wherein power transmission from an RF generator (or other energy source) to the electrodes 294 is controlled by a foot switch. As shown by way of example in FIGURE 10d, and in accordance with one embodiment of a present invention, a handle 312' is provided with a manually operable on-off switch 313. On-off switch 313 allows the physician to selectively enable and disable the supply of RF ablation energy (and other types of power) to the electrode(s) on the distal portion of the probe.

In addition to the global on-off switch 313, the exemplary handle 312" shown in FIGURE 10e also includes a plurality of individual on-off switches 315 for each of the electrodes. The individual on-off switches 315 allow the physician to selectively control the supply of power to individual electrodes. The exemplary handle 312", which has seven individual on-off switches 315, is preferably used in a probe having seven electrodes. If for example, the physician intends to ablate tissue with only three of the electrodes, then the three chosen electrodes may be enabled by way of the corresponding switches 315 prior to placing the global on-off switch 313 in the "on" position.

A plurality of indicator elements 317 are also provided on the exemplary handle 312" shown in FIGURE 10e. Preferably, there is one indicator element 317 for each of the on-off switches 315. In the illustrated embodiment, the indicator elements 317 are in the form of buttons that are raised when a corresponding on-off switch 315 is depressed. This provides the physician with a tactile as well as visual indication of the on-off status of the switches 315. The indicator elements 317 may also be in the form of indicator lights. Sound-based indications of the on-off status of the switches 315 may also be used. For example, a speaker on the handle or the power supply device may be employed to periodically indicate which of the switches 315 are in the "on" position.

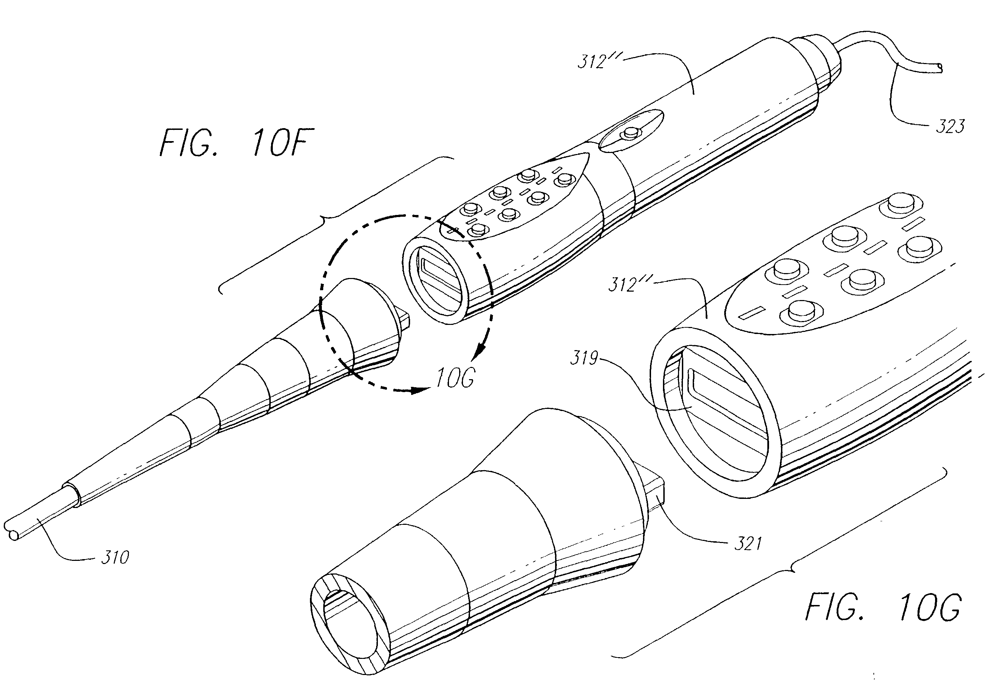

In accordance with another aspect of the present invention, a probe may be configured such that the handle is re-usable and the remaining portions of the probe are disposable or separately re-usable. Turning to FIGURES 10f and 10g, exemplary handle 312" includes an edge-type connector having a first portion 319 on the handle and a second portion 321 on the remaining portion of the probe. In the illustrated embodiment, the remaining portion is primarily the shaft 310 which, as described above, supports a plurality of electrode elements (not shown).

The first and second connector portions 319 and 321 have elements that will mechanically couple the handle to the remaining portion of the probe and release the two when desired. The first and second connector portions will also connect signal wires from the electrodes (or other operative elements) and temperature sensors to the energy source. A locking mechanism (not shown) may be used to maintain the integrity of the connection between the two connector portions. A cable 323 may be provided to connect the handle to an energy source.

The handles shown in FIGURES 10e-10g may be used with any of the probes disclosed herein and the features of such handles may be incorporated into any of the other handles disclosed herein.

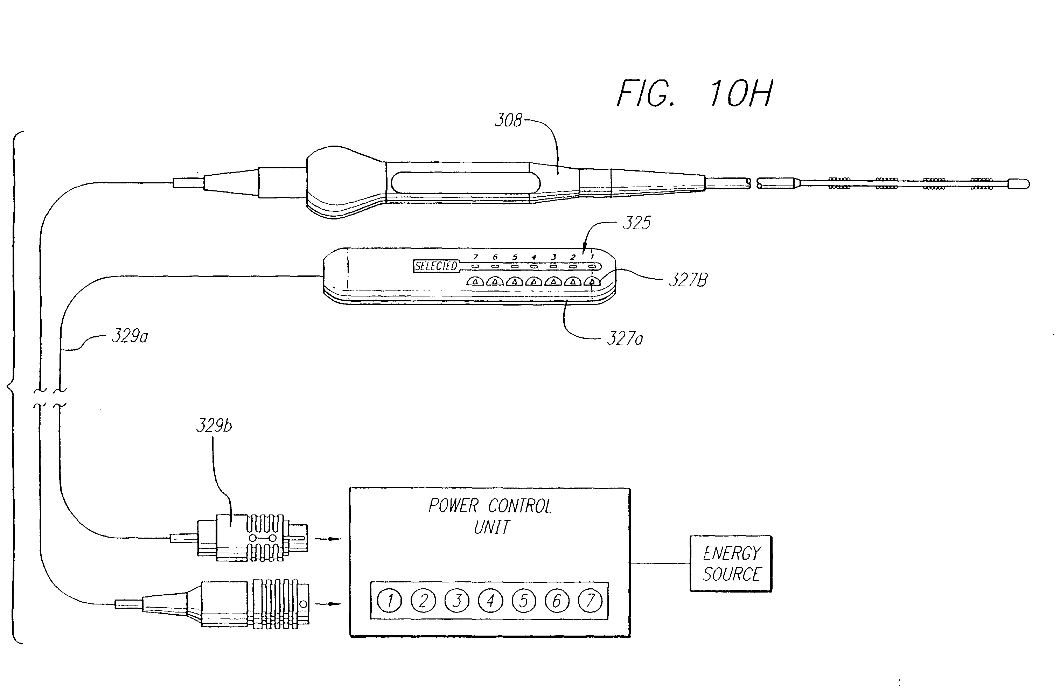

As shown by way of example in FIGURES 10h and 10i, and in accordance with one embodiment of a present invention, a remote power control unit 325 may be used in conjunction with a surgical probe 308 or a catheter (not shown). The remote power control unit 325 includes a main body 327a and a plurality of on/off switches 327b. Preferably, there is one on/off switch 327b for each electrode and, in the illustrated embodiment, there are seven electrodes and seven on/off switches. The remote power control unit can also include a global power on/off switch (not shown). Alternatively, a foot pedal (not shown) may be provided to perform the same function.

The size and shape of the remote power control unit 325 allow it to be easily grasped in one hand by the physician or other member of the operating room staff. Preferably, the remote power control unit 325 is about 8 inches (20.3 cm) in length, about 1.5 inches (3.8 cm) in width and about 0.5 inch (1.3 cm) in thickness. Of course, the size and shape can be adjusted to suit particular needs.

The remote power control unit 325 may be used in conjunction with conventional electrophysiology power control units, such as that shown in U.S. Patent No. 5,545,193, that are connected to a source of energy (such as ablation energy) and provide individual electrode control. To facilitate such use, the remote control device includes a connection apparatus which, in the illustrated embodiment, consists of a cable 329a and a connector 329b. The cable 329a should be relatively long, i.e. between about 6 feet (1.8 m) and about 15 feet (4.6 m) in length and is preferably 10 feet (3 m). The connection apparatus can also be in the form of a wireless transmitter/receiver arrangement or any other suitable device. The surgical probe 308 is also connected to the electrophysiology power control unit. When a foot pedal is used, it too is connected to the electrophysiology power control unit.

The exemplary remote power control unit 325 includes indicia 333 in the shape of the distal portion of a surgical probe, indicator lights 335, and numbers corresponding to the respective electrodes on the probe. The combination of indicia, lights and numbers allows the physician to readily determine which electrodes are enabled and which electrodes are disabled.

The surgical probe 308 (as well as the other probes disclosed herein) and the remote power control unit 325 are sterilizable. To that end, these devices are either entirely hermetically sealed or selected portions, such as those enclosing electronic components, are sealed. Those components which are not sealed are penetrable by a gas sterilant, such as ethylene oxide (EtO). The surgical probes and remote power control units should also be splash-proof.

In those instances where a malleable shaft 310 is desired, the hypo-tube 316 may be the heat treated malleable hypo-tube 316 shown in FIGURES 10a, 12 and 13. By selectively heat treating certain portions of the hypo-tube, one section of the hypo-tube (preferably the distal section) can be made more malleable than the other. This will alleviate any discontinuity between the distal section 314 and the shaft 310 when the distal section is malleable.

A plurality of temperature sensing elements (such as thermocouples which are not shown) may be located on, under, abutting the longitudinal end edges of, or in between, the electrode elements 294 in any of the exemplary devices disclosed herein. Additionally, a reference temperature sensing element may be provided. For example, a reference temperature sensing 324 may be located in the handle so that room temperature will be used as the reference as shown in FIGURE 10a. The reference temperature sensor may, alternatively, be provided on or near the distal tip of the device. Another alternative is to use an electronic circuit to function as the reference temperature sensor. A reference temperature sensor can also be placed on the patient or in the operating room and the physician can simply input the reference temperature into the power control device. It should be noted that the accuracy of the reference temperature sensor is less important in applications where the patient is on bypass because the convective cooling effects of blood flowing past the electrodes is substantially reduced. Also, the present surgical devices provide better tissue contact than conventional catheter-based devices, which provides more accurate temperature monitoring.

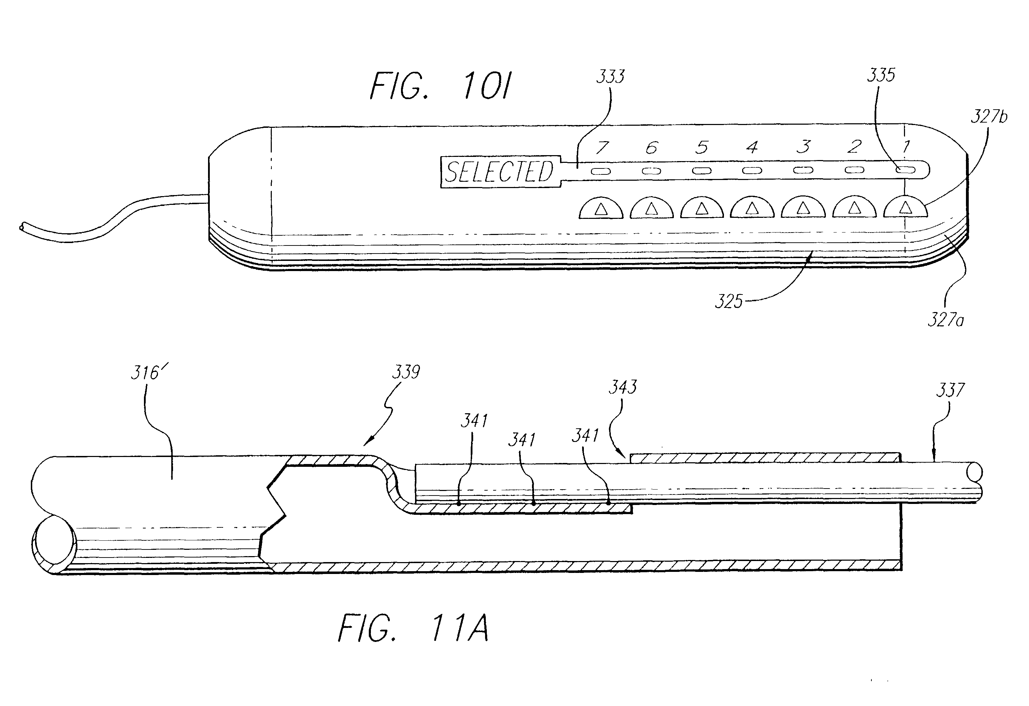

The distal section 314 can be either somewhat flexible, in that it will conform to a surface against which it is pressed and then spring back to its original shape when removed from the surface or, as noted above, malleable. A bending modulus of between 3 lb.-in.$^2$ (86 N-cm$^2$) and 50 lb.-in.$^2$ (1435 N-cm$^2$) is preferred. As shown by way of example in FIGURE 11a, a somewhat flexible distal section 314 may include a spring member 330, which is preferably either a solid flat wire spring (as shown), a round wire, or a three leaf flat wire Nitinol spring, that is connected to the distal end of the hypo-tube 316. Other spring members, formed from materials such as 17-7 or carpenter's steel, may also be used. A series of lead wires 332 and 334 connect the electrode elements 294 and temperature sensor elements, respectively, to the PC board 320. The spring member 330 and leads wires 332 and 334 are enclosed in a flexible body 336, preferably formed from PEBAX® material, polyurethane, or other suitable materials. The spring member 330 may also be pre-stressed so that the distal tip is pre-bent in the manner shown in FIGURE 10a. Also, an insulating sleeve 331 may be placed between the spring member 330 and the lead wires 332 and 334.

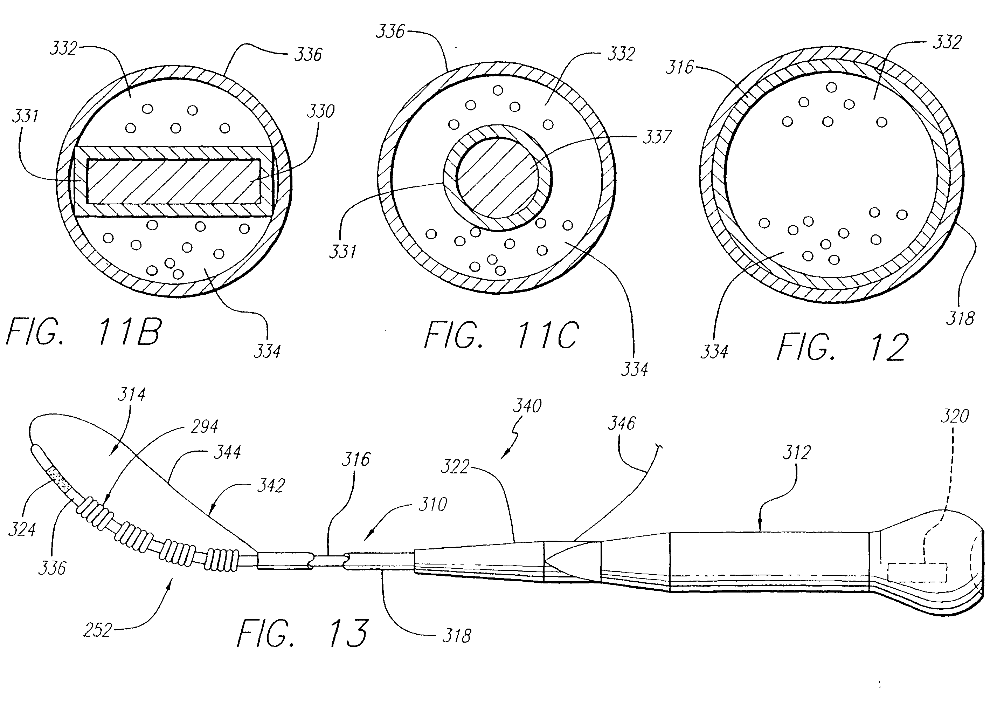

In those instances where a malleable distal portion 314 is desired, the spring member 330 may be replaced by a mandrel 337 made of suitably malleable material such as annealed stainless steel or beryllium copper, as illustrated for example in FIGURE 11b. The mandrel will ideally be fixed to the distal tip of the device (by, for example, soldering, spot welding or adhesives) and run through the shaft into the handle where it will also be fixed to insure good torque transmission and stability of the distal tip. Alternatively, the malleable mandrel may be fixed directly within the distal end of the shaft's hypo-tube 316 and secured by, for example, soldering, spot welding or adhesives.

Alternatively, and as shown by way of example in FIGURE 11c, a slot 339 may be formed in the hypo-tube 316'. The malleable mandrel 337 is inserted into the slot 339 and then held in place by spot welds 341 (shown), solder or adhesive. The slot 339 includes an opening 341 at one end thereof through which the mandrel 337 extends. The slot 339 could also include another opening at the other end. The slot 339 is located in spaced relation to the proximal end of the hypotube 316' to create additional support for the mandrel 337 when it is bent and formed into various shapes. By shortening the length of the mandrel 337, the torque of the shaped distal assembly is increased relative to the embodiment described above wherein the mandrel is anchored within the handle.

The distal portion 314 may also be formed by a hypo-tube that is simply a continuation of the shaft hypo-tube 316. However, the distal end hypo-tube can be a separate element connected to the shaft hypo-tube 316, if it is desired that the distal end hypo-tube have different stiffness (or bending) properties than the shaft hypo-tube.

The shaft 310 may be from 4 inches to 18 inches (10.2 cm to 45.7 cm) in length and is preferably 6 to 8 inches (15.2 to 20.3 cm). The distal portion 314 may be from 1 to 10 inches (2.5 cm to 25.4 cm) in length and is preferably 2 to 3 inches (5.1 to 7.6 cm). To facilitate the formation of long continuous lesions, the distal portion 314 preferably includes six spaced electrode elements 294 that are approximately 12mm in length. The number and length of the electrode elements 294 can, of course, be varied to suit particular applications.

In accordance with some embodiments of this invention, and as shown by way of example in FIGURES 10b and 10c, the distal section 314 may be provided with a distal (or tip) electrode. Referring first to FIGURE 10b, the distal electrode 326 may be a solid electrode with a through hole for one or more temperature sensors. Another exemplary electrode is the shell electrode 328 shown in FIGURE 10c, which could also have one or more temperature sensors inside. The distal electrodes have a variety of applications. For example, a distal electrode may be dragged along an anatomical surface to create a long lesion. The distal electrode may also be used to touch up lesions (straight or curvilinear) created by electrode elements 294 if, for example, the distal section 314 does not exactly conform to the anatomical surface, and to continue lesions formed by the electrode elements. The distal electrode may also be used to create lesions in anatomical ridges that are shaped such that the integrity of the surgical device would be compromised if the distal section 314 were bent to conform to the ridge.

As shown by way of example in FIGURE 13, an exemplary surgical probe 340 is provided with a pull wire 342 that allows the physician to adjust the curvature of the distal portion 314 from no curve, to a slight curve, an extreme curve, or even a loop, as desired. The pull wire distal portion 344 is connected to the distal tip of distal section 314. The distal portion of the pull wire enters the shaft proximal to the ablation electrodes, and the proximal portion 346 exits through an aperture formed in the handle 312. But for the pull wire 342, the probe 340 is substantially the same as the spring tip probe version shown in FIGURES 10a and 11a. Alternatively, the proximal portion of the pull wire 342 may be associated with a handle/knob arrangement such as that shown in FIGURE 9f.

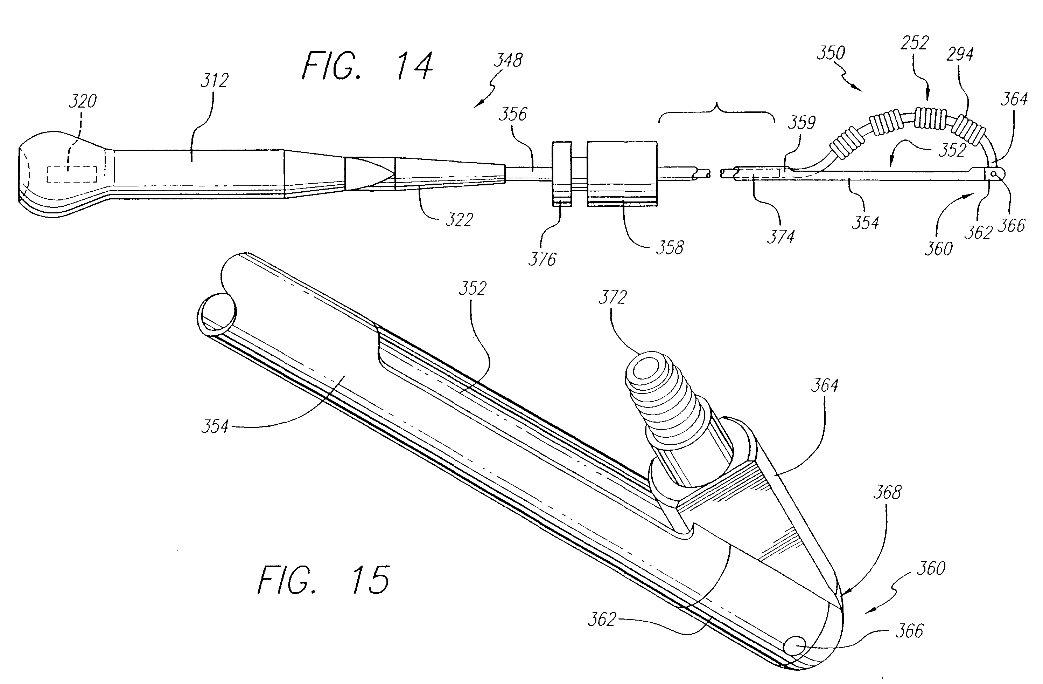

In accordance with another embodiment of this invention, and as illustrated for example in FIGURES 14 and 15, a surgical probe 348 is provided with a distal loop structure 350 that includes an operative element 252 in the form of a plurality of electrodes 294. The distal loop structure 350, which extends through an opening 352 in a sheath 354, is connected to a shaft 356. The shaft is, in turn, connected to the handle 312. The proximal portion of the sheath 354 includes a handle 358 that allows the sheath to be moved distally and proximally. The stiffness of the loop structure 350 is less than that of the sheath 354. As such, when the sheath 354 is pulled in the proximal direction, the loop structure 350 will bulge out of the sheath opening 352 in the manner shown in FIGURE 14. When the sheath 354 is returned to its distal most position, the loop structure 350 will slide back into the sheath such that the sheath and the loop structure are coaxial.

The exemplary loop structure 350 is similar to the distal portion 314 of the probe shown in FIGURES 10a and 11a in that it includes a spring member (not shown), such as a leaf spring or a flat wire spring (preferably formed from Nitinol), which is covered by a flexible material such as a PEBAX® tube 359. In addition to allowing the distal portion 350 to bulge outwardly, the spring member can be flat so that it also provides resilience which helps the distal portion conform to the anatomical surface of interest and prevents "out of plane bending."

In the exemplary embodiment illustrated in FIGURES 14 and 15, a pivot assembly 360 is provided on the distal end of the sheath 354. The pivot assembly 360 includes a base member 362 and a pivot member 364 which is secured to the base member by a pivot pin 366. Referring more specifically to FIGURE 15, the pivot member 364 pivots within a slot 368 that is formed in the base member 362. The size and shape of the slot 368, and the location of the pivot member 364 therein, may be adjusted to adjust the shape of the loop. For example, the location of the pivot member 364 and the shape and size of the slot 368 may be varied such that the pivot member can only rotate 30, 60, 90 or 180°. However, up to 270° of rotation is possible. The pivot member 364 includes a connector 372 (such as the illustrated threaded or barbed connector) for securing the distal end of the loop structure 350 to the pivot member.

The rigidity, malleability, or flexibility of the probe 348 may be provided in a number of ways. For example, the sheath 354 may be formed from a rigid stainless steel hypo-tube, a relatively stiff somewhat flexible stainless steel hypotube, or a relatively stiff malleable annealed stainless steel hypo-tube. Additionally, or alternatively, the shaft 356 may be a rigid (or somewhat flexible) stainless steel hypo-tube or a malleable annealed stainless steel hypo-tube. In either case, the distal end 374 of the shaft 356 will abut the flexible portion of the loop structure 350. Other materials can, of course, be used in place of stainless steel. A rigid high durometer plastic tube, for example, may be substituted for the stainless steel hypo-tube in the sheath or shaft.

Once the sheath 354 and shaft 356 are positioned relative to one another such that the desired loop is produced, the sheath may be secured to the shaft by a touhy borst connector 376 that is secured to the distal end of the sheath 354 between the handle 358 and the handle 312.

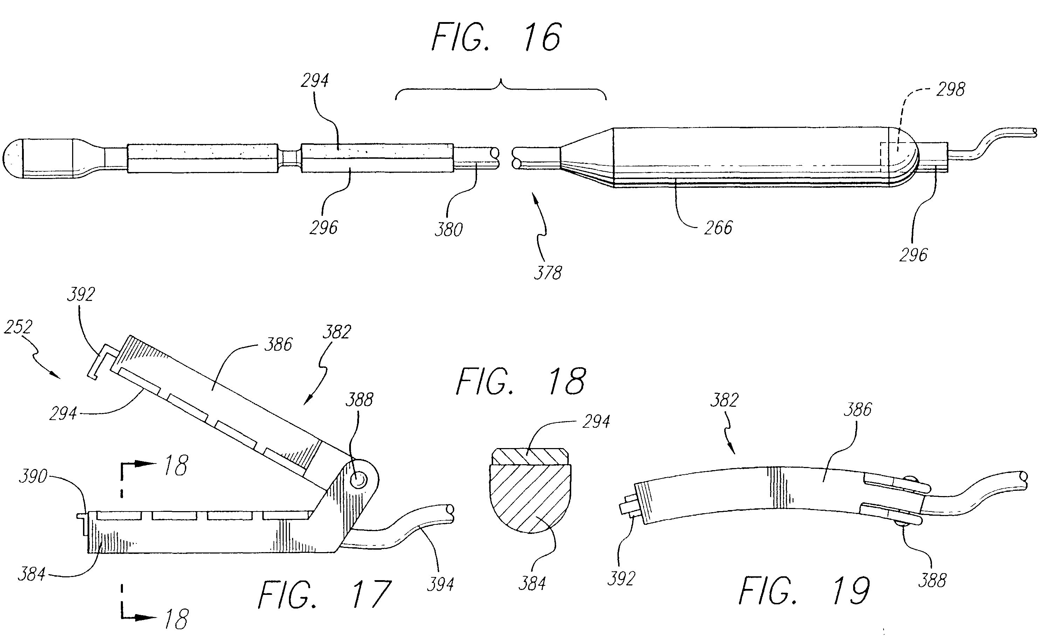

An ablation probe 378 in accordance with another aspect of this invention is illustrated, for example, in FIGURE 16. The probe includes a shaft 380 (similar to shafts 254, 310 or 356 described above) on which one or more ablation electrodes 294 are mounted. As described in greater detail in Section II below, masking 296 may be used to control the focus of the ablation energy and/or prevent convective cooling when the probe is in the blood pool. A handle 266 is also provided. The shaft 380 is preferably between approximately 4 to 16 inches (10.1 and 40.6 cm) in length, between approximately 3 and 8 mm in diameter. Additionally, the shaft may either be rigid or relatively stiff and, if relatively stiff, can be either malleable or somewhat flexible. The ablation probe 378 may be used for a variety of procedures. For example, the shaft may be inserted into the heart to perform ablation procedures.

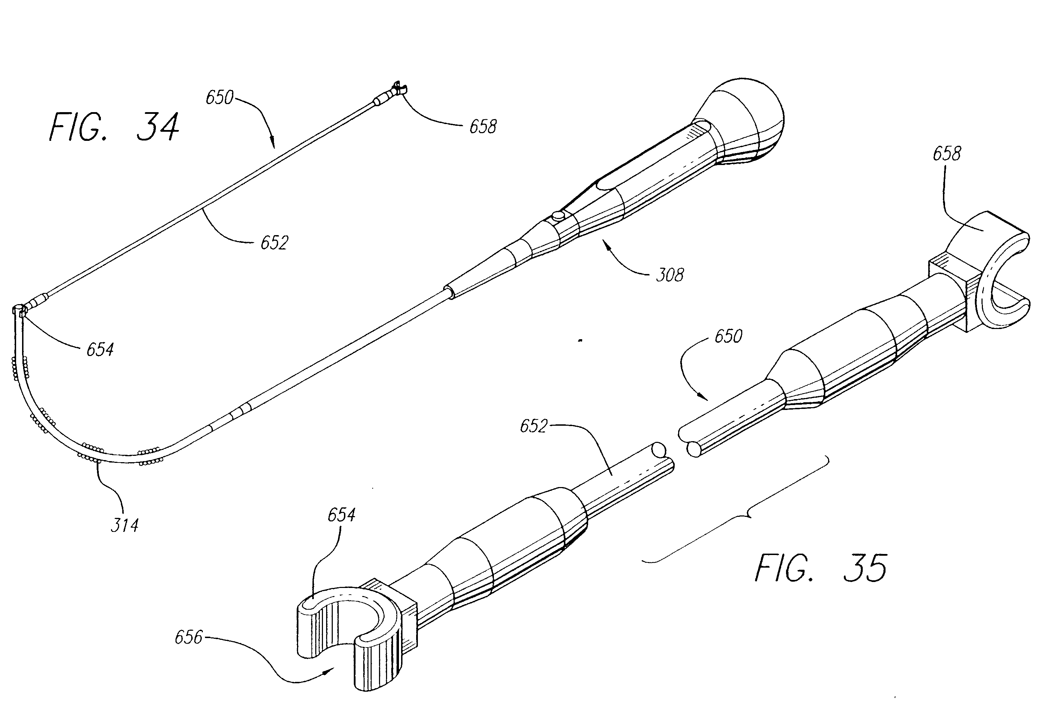

Turning to FIGURES 34 and 35, a pressure application probe 650 may be used to apply pressure to the distal section of a probe, such as the probe 308 shown in FIGURE 10a, or any other operative element supporting device. The application of pressure with the probe 650 can improve the level of contact between tissue and, for example, the distal section 314 of the probe 308. The pressure application probe 650 includes an elongate main body portion 652 and at least one engagement device 654. The exemplary pressure application probe shown in FIGURES 34 and 35 also includes a second engagement device 658. As discussed in detail below, the second engagement device 658 has a slightly different shape than the engagement device 654.

The main body portion 652 is preferably either rigid, malleable or somewhat flexible and about 4 to 18 inches (10.2 cm to about 45.7 cm) in length, although the length may be adjusted to suit particular applications. When a malleable main body portion is desired, the main body portion 652 may be formed in the manner described above with respect to the shaft 254, and preferably consists of a soft metal rod or tube, or a settable plastic rod or tube. For example, the shaft 254 may be formed from a nickel titanium rod or tube, which is ductile at room temperature and which will straighten out at elevated temperatures such as those used during autoclave sterilization. Regardless of stiffness, the outer surface of the main body portion 652 should be covered with insulating material such as PEBAX® or urethane. The engagement device 654 is preferably formed from insulating material such as polycarbonate, urethane, glass filled thermoplastic or ABS.

The engagement device may have any of a variety of configurations. In the exemplary embodiment illustrated in FIGURES 34 and 35, the engagement devices 654 and 658 are generally c-shaped, with engagement device 658 having a more open shape. In use, the c-shape helps maintain the engagement devices at the desired location on the distal portion of the surgical probe 308 so that pressure can be applied to the desired location. The open shape of the engagement device 658 allows the engagement device to be readily repositioned along the distal portion of the surgical probe without disturbing the position of the surgical probe relative to the tissue.

The c-shaped engagement device 654 can be coupled to the distal section 314 of the probe 308 as shown in FIGURE 34, or any other probe, by either inserting the distal tip of the probe through the opening 656 or by snap-fitting the engagement device 654 over the distal section. When snap-fitting is desired, the engagement device should be somewhat flexible. This arrangement allows the pressure application probe 650 to be rotated relative to the probe 308 when the two are engaged. As a result, the pressure application probe 650 can be reoriented without moving the probe 308.

The pressure application probe 650 may also be used to move the probe 308 within the patient when the two are engaged.

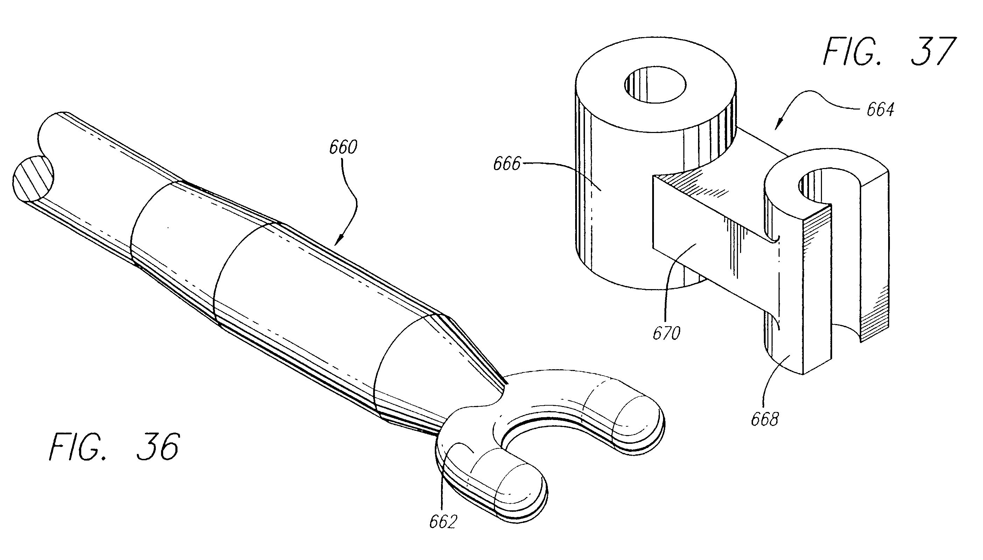

As shown by way of example in FIGURE 36, an exemplary pressure application probe 660 is provided with an engagement device 662 having a relatively narrow profile. The narrow profile allows the probe 660 to engage the distal section of an operative element supporting device, such as the distal section 314 of probe 308, even when the two devices are oriented at severe angles relative to one another. Of course, the engagement device is not limited to the shapes shown in FIGURES 34-36. Any shape that is capable of engaging the distal portion of a probe may be used.

Although not limited to such a use, the pressure application probes shown in FIGURES 34-36 are especially useful in thoroscopic procedures. Here, the pressure application probe may be inserted into a patient through one port, while the electrode supporting probe is inserted through another port and connected to the pressure application probe.

Another device which may be used in conjunction with probes such as the probe 308 shown in FIGURE 10a is illustrated, for example, in FIGURE 37. The exemplary coupling device 664 includes a base member 666, a generally c-shaped engagement device 668 (similar to that described above) and, in the illustrated embodiment, a connecting member 670. The base member 666 and engagement device 668 can also be directly connected to one another.

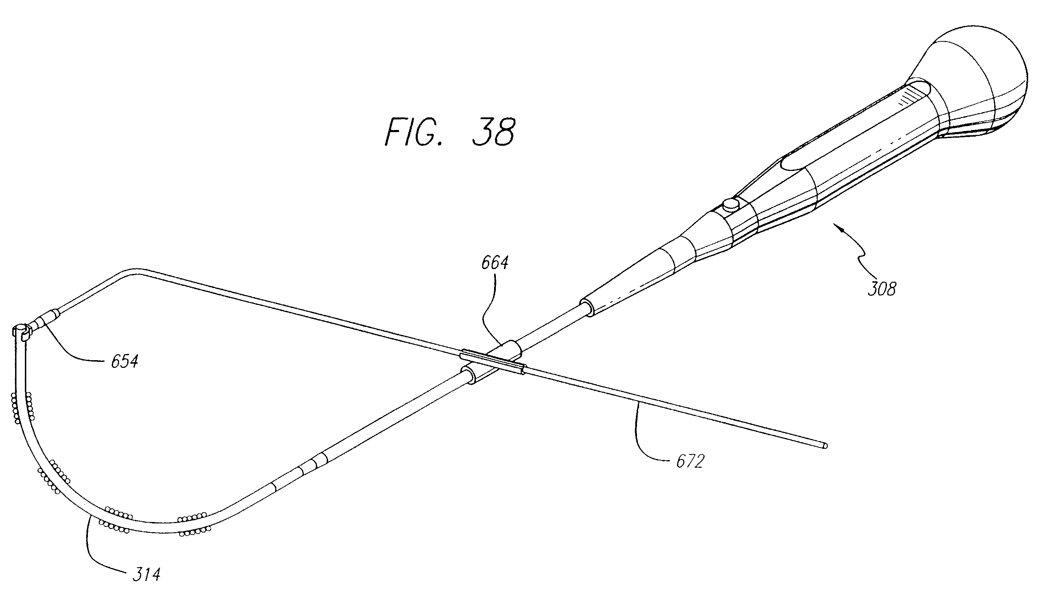

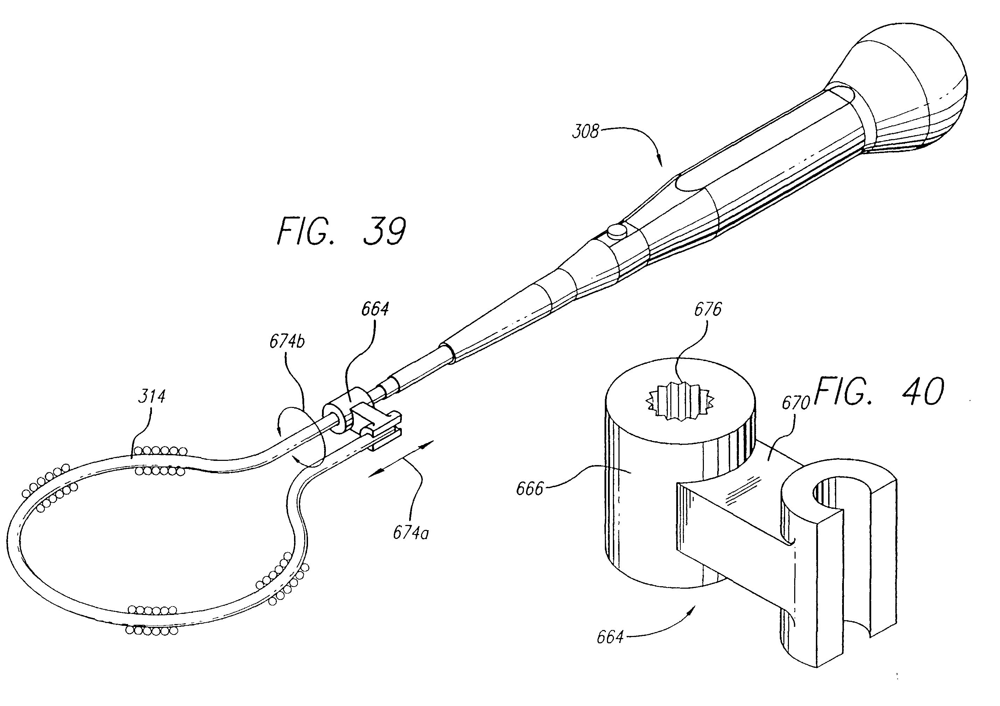

The coupling device 664 has a wide range of uses. For example, the coupling device may part of a pressure application probe 672, as shown in FIGURE 38. Another exemplary use of the coupling device 664 is shown in FIGURE 39. Here, the coupling device 664 is placed on a probe such as probe 308 and used to create a distal loop. The coupling device can be located at different points along the length of the probe and arranged at different rotational orientations relative to the probe (note arrows 674a and 674b) in order to control the shape of the loop. To that end, the base member 666 and a portion of the distal section 314 can include respective sets of teeth that allow the rotational orientation of the coupling device 664 to be fixed relative to the probe 308. Note teeth 676 in FIGURE 40.

In order to increase the number of coupling device applications, the connecting member 670 may be configured in a variety of ways. For example, the connecting member 670 can be rigid, flexible, somewhat flexible, or malleable. The connecting member 670 can also be in the form of a swivel or pivot. The base member 666 and engagement device 668 can also be fixed at various angles relative to one another (note, for example, FIGURE 38).

## II. The Operative Elements

## A. Exemplary Operative Elements

In the exemplary embodiments illustrated in FIGURES 1-16, the operative element 252 is made up of a plurality of electrode elements 294. Electrode elements 294 can serve a variety of different purposes. The operative elements 252 may also be lumens for chemical ablation, laser arrays, ultrasonic transducers, microwave electrodes, and D.C. hot wires.

In the illustrated embodiments, the principal use of the electrode elements is to transmit electrical energy and, more particularly, RF energy, to ablate heart tissue. However, the electrode elements can also be used to sense electrical events in heart tissue. Alternatively, or in addition, the electrode elements can serve to transmit electrical pulses to measure the impedance of heart tissue, to pace heart tissue, or to assess tissue contact using conventional pacing and sensing techniques. Once the physician establishes contact with tissue in the desired heart region, the physician applies ablating energy to the electrode elements.

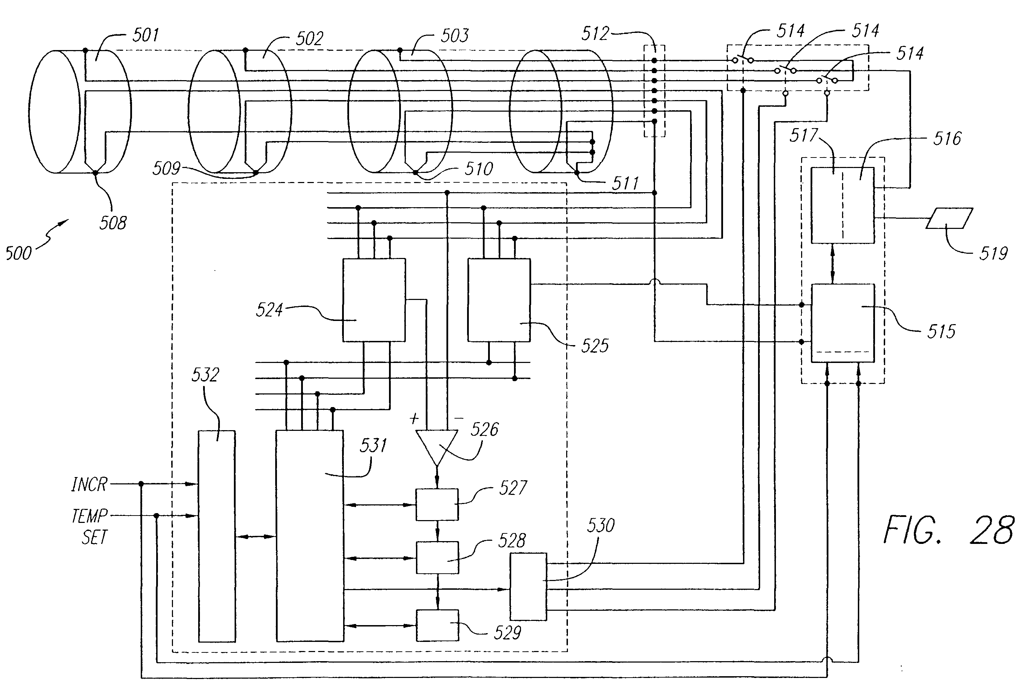

In the exemplary embodiments illustrated in FIGURES 1-16, the electrode elements 294 are electrically coupled to individual wires (see reference numeral 295 FIGURES 8b and 9e and reference numeral 332 in FIGURES 11a, 11b and 12) to conduct ablating energy to them. The wires are passed in conventional fashion through a lumen extending through one of the spline legs and the shaft 254 into a PC board in the handle 266, where they are electrically coupled to a connector 296 which is received in a port 298 (see FIGURE 1). The connector 296 plugs into a source of RF ablation energy. A plurality of temperature sensing elements (not shown), such as theremocouples or thermistors, may also be provided on the spline assemblies shown herein. Such temperature sensing elements may be located on, under, abutting the longitudinal end edges of, or in between, the electrode elements 294. For temperature control purposes, signals from the temperature sensor elements are transmitted to the source of ablation energy by way of wires (see reference numeral 297 in FIGURES 8b and 9e and reference numeral 334 in FIGURES 11a, 11b and 12) which are also connected to the PC board. Suitable temperature sensor elements and controllers which control power to an electrode based on a sensed temperature are disclosed in U.S. Patent Nos. 5,456,682 and 5,582,609. The respective numbers of wires will, of course, depend on the numbers of sensors and electrodes used in a particular application. A suitable temperature control system is described below with reference to FIGURES 28-31.

The electrode elements can be assembled in various ways. They can, for example, comprise multiple, generally rigid electrode elements arranged in a spaced apart, segmented relationship. The segmented electrodes can each comprise solid rings of conductive material, like platinum, which makes an interference fit about the annular spline member. Alternatively, the electrode segments can comprise a conductive material, like platinum-iridium or gold, coated upon the device using conventional coating techniques or an ion beam assisted deposition (IBAD) process. For better adherence, an undercoating of nickel or titanium can be applied. The electrodes can also be in the form of helical ribbons.

Alternatively, the electrode elements can comprise spaced apart lengths of closely wound, spiral coils wrapped about the device to form an array of generally flexible electrode elements. The coils are made of electrically conducting material, like copper alloy, platinum, or stainless steel, or compositions such as drawn-filled tubing (e.g. a copper core with a platinum jacket). The electrically conducting material of the coils can be further coated with platinum-iridium or gold to improve its conduction properties and biocompatibility.

Electrode elements can be formed with a conductive ink compound that is pad printed onto a nonconductive tubular body. A preferred conductive ink compound is a silver-based flexible adhesive conductive ink (polyurethane binder), however other metal-based adhesive conductive inks such as platinum-based, gold-based, copper-based, etc., may also be used to form electrodes. Such inks are more flexible than epoxy-based inks.

As illustrated for example in FIGURE 7, the electrode elements can also include a porous material coating 299, which transmits ablation energy through an electrified ionic medium. For example, electrode elements and temperature sensor elements may be coated with regenerated cellulose, hydrogel or plastic having electrically conductive components. With respect to regenerated cellulose, the coating acts as a mechanical barrier between the surgical device components, such as electrodes, preventing ingress of blood cells, infectious agents, such as viruses and bacteria, and large biological molecules such as proteins, while providing electrical contact to the human body. The regenerated cellulose coating also acts as a biocompatible barrier between the device components and the human body, whereby the components can now be made from materials that are somewhat toxic (such as silver or copper).

For applications in which the ablation electrode is in contact with flowing blood as well as tissue, such as when the patient is not on bypass, coating electrodes with regenerated cellulose decreases the effect of convective cooling on the electrode because regenerated cellulose is a poor thermal conductor as compared to metal. Thus, the effect of convective cooling by blood flowing past the regenerated cellulose coated electrodes is diminished. This provides better control for a lesion-generating process because the hottest tissue temperature is closer to the ablation electrode.

Furthermore, the regenerated cellulose coating decreases the edge effects attributed to delivering RF energy to an electrode having a sharp transition between the conductive electrode and insulating material. The current density along the electrode and power density within tissue are more uniform, which reduces the incidence and severity of char and/or coagulum formation. The more uniform current density along the axis of the device also results in a more uniform temperature distribution at the electrode, which decreases the requirement for precise placements of the temperature sensors at the ablation electrodes. Additionally, by coating a device with regenerated cellulose to create the outer surface, less labor-intensive methods of forming electrodes and bonding wires to electrode surfaces can be used.

During the coating process, a device such as the one of the above-described distal spline assemblies is coated with a viscose solution. The viscose solution is preferably cellulose xanthate, which is a form of solubilized cellulose derivative that is dissolved in a sodium hydroxide solution. The viscose solution is dip-coated onto the distal end assembly, which includes the electrodes, signal wires, temperature sensors, etc. The coated device is then regenerated by contacting it with an acid, such as sulfuric acid, which converts the xanthate back into the cellulose structure. The term regenerated cellulose refers to cellulose which has been converted from a solubilized cellulose derivative back into a pure cellulose structure. This regeneration process creates large enough micro size pores in the coating allowing ionic transport yet small enough to prevent ingress of blood cells, infectious agents, such as viruses and bacteria, and large biological molecules such as proteins.

Once the cellulose is regenerated, it is rinsed with water to remove acid residuals and sulfur compounds. An oxidizing agent (bleach, etc.) may be added to the rinse water to accelerate the removal of sulfur compounds. After the cellulose is regenerated, it is fully cured in an environmental chamber at a low humidity. Thereafter, it is preferable to make the regenerated cellulose flexible when dry, and to do so moisture is reintroduced into the cellulose coating material by setting the environmental chamber to a higher humidity. Alternatively, a small quantity of a material such as glycerol may be applied to the coating, and the hydroscopic nature of the glycerol will hydrate the cellulose coating to create sufficient flexibility. An overall thickness range for operable regenerated cellulose coatings is from 0.001 inch to 0.015 inch (0.25 mm to 3.8 mm), with a preferable thickness range being from 0.001 inch to 0.003 inch (0.25 mm to 0.76 mm); a preferred thickness being approximately 0.002 inch (0.51 mm).

Materials other than regenerated cellulose that are mechanically robust and that have suitable characteristics could be used for the coating material. Hydrophilic materials that have effective pore sizes from 500 to 500,000 Daltons with a porosity of 1-10% and which are biocompatible could be effective. Some types of hydrogels, such as those used for disposable contact lenses are good candidate materials. Plastic materials that have additives to make them semiconductive could also be used. The loaded plastic would need to have a resistivity in the range of about 200-2,000 ohm-cm, and would need to be applicable in very thin films to the device.

The thickness of the cellulose coating is controlled by the viscosity of the coating solution and the dipping rate, and a different viscosity of the coating solution can be achieved by diluting it with the sodium hydroxide solution. A variable wall thickness can be achieved by varying the extraction rate during the dipping process. The slower the extraction rate, the thinner the wall thickness, and the faster the extraction rate, the thicker the wall thickness. An increased coating wall thickness can also be obtained by multiple layers of coating. To ensure proper lamination between such layers, each layer is coagulated with a salt solution (sodium sulfate, etc.) before applying another layer. In addition, spraying and co-extruding the viscose solution over the electrodes and the distal section can also be used to achieve a variable wall thickness cellulose coating.

In another method for covering a distal electrode assembly, a tubular casing of regenerated cellulose material is created on a mandrel. The regenerated cellulose casing is then shrunk onto the distal assembly.

The regenerated cellulose coating may also be applied over a "wet" electrode element. The moisture from the wet electrode element prevents the electrode elements from sticking to tissue during an ablation procedure. A wet electrode element is formed by a material that has high absorption capacity for liquids, such as an open cell sponge, hydrogel or cloth. Alternatively, the regenerated cellulose coating may simply be wet prior to the procedure, such as an ablation procedure.

The electrode elements may be operated in a uni-polar mode, in which the ablation energy emitted by the electrode elements is returned through an indifferent patch electrode (not shown) externally attached to the skin of the patient. Alternatively, the elements may be operated in a bi-polar mode, in which ablation energy emitted by one or more electrode elements is returned through other electrode elements. The amount of power required to ablate tissue ranges from 5 to 150 W.

The electrode elements are preferably about 4 mm to about 20 mm in length. Continuous lesion patterns uniformly result when adjacent electrode elements are spaced no farther than about 2.5 times the electrode segment diameter apart. Further details of the formation of continuous, long and thin lesion patterns are found in PCT Publication No. WO 95/10318, entitled "Systems and Methods for Forming Elongated Lesion Patterns in Body Tissue Using Straight or Curvilinear Electrode Elements." Similar sizing and spacing may be used in conjunction with the other embodiments illustrated herein.

Using rigid electrode segments, the length of the each electrode segment can vary from about 2 mm to about 10 mm. Using multiple rigid electrode segments longer than about 10 mm each adversely effects the overall flexibility of the element. Generally speaking, adjacent rigid electrode segments having lengths of less than about 2 mm do not consistently form the desired continuous lesion patterns.

When flexible electrode segments are used, electrode segments longer that about 10 mm in length can be used. Flexible electrode segments can be as long as 50 mm. If desired, the flexible electrode structure can extend uninterrupted along the entire length of a support spline.

### B.    Operative Element Considerations in a Non-Convective Cooling Environment

In the exemplary embodiments shown in, for example, FIGURES 1-6a, 7, 9a-f, 10, 13 and 14, the electrode elements are not masked. Such embodiments are particularly useful when little to no fluid flow will be present, such as when the heart is on bypass and there is no blood flow within the heart. Here, air acts as an insulator and produces only modest convective cooling effects, as compared to a flowing blood pool that has a higher convection coefficient than virtually static air. Energy transmission is, therefore, essentially limited to the RF energy that is transmitted from the portion of the electrode surface that is in contact with the tissue to either a ground electrode, or another electrode within the group of electrode elements. The overall impedance of the system will increase (as compared to a situation where blood is present) due to the smaller effective surface area between the electrode and tissue.

Both of these conditions, focused RF energy and low heat dissipation into the air, will impact the ablation because they result in a high current density with high local desposition of heat without the heat sinking that convective cooling provides. When creating long lesions with a conventional catheter, char can be created as the tip is dragged because of the high current density and the difficulty in monitoring tissue temperature and controlling power that is inherent in the dragging process. The present invention, however, can take advantage of the high current density because the electrodes are not being dragged. For example, a number of electrodes can be used to ablate simultaneously because the effective (tissue contacting) surface area between all of the ablating electrodes is smaller and the convective cooling effects are reduced, as compared to situations where blood is present. This reduces the power requirements of the system. In addition, by using electrodes with lower thermal mass (as compared to a conventional solid tip electrode), less heat will be retained by the electrode and better temperature sensing can be made at the tissue surface. This will speed up the creation of the lesions and enable better lesion creation control.

It is also noteworthy that the masking described in the following section can be useful during bypass because tissue can partially wrap around the electrodes when the distal end of the device is pressed against the tissue. Such masking can also be used to control lesion thickness.

### C.    Operative Element Considerations in a Convective Cooling Environment

In instances where the patient will not be on bypass and blood will be flowing past the electrodes, or in other situations when fluid flow is present, the portion of the electrode elements (or other operative elements) not intended to contact tissue may be masked through a variety of techniques with a material that is preferably electrically and thermally insulating. For example, a layer of UV adhesive (or another adhesive) may be painted on preselected portions of the electrode elements to insulate the portions of the elements not intended to contact tissue. Alternatively, a slotted sheath may be positioned over the portion of the electrode elements not intended to contact tissue. Deposition techniques may also be implemented to position a conductive surface only on those portions of the spline assembly intended to contact tissue. A coating may be formed by dipping the electrode elements in polytetrafluoroethylene (PTFE) material.

As shown by way of example in FIGURE 8a, a polymer layer 296 may be thermally fused over the electrodes 294 to mask desired portions of the electrodes. The layer prevents the transmission of ablating energy directly into the blood pool and directs the applied ablating energy directly toward and into the tissue.

An exemplary process for applying the polymer layer is as follows. A segment of shaft tubing is cut long enough to cover the desired electrodes, and is then split in half (or other desired angle) along the axis. One half is placed over the assembled distal section so that it covers the side of the electrodes that are to be masked. A piece of polymeric shrink tubing, preferably RNF-100 or irradiated LDPE, is then carefully slid over the catheter distal end, so that the mask tubing is not moved from its placement over the electrodes and so that it stops approximately 2 cm beyond the end of the tubing half. The distal end is then heated in a controlled heat source at approximately 400°F (204°C) so that the mask tubing fuses into the distal shaft tubing along its length, and so that all of its edges are well fused into the shaft tubing, but not fused so much that the covered electrodes begin to poke through. Finally, the polymeric shrink tubing is split on one end and the assembly is heated at approximately 225°F (107°C) while the polymeric shrink tubing is slowly peeled off of the fused catheter shaft.

Additionally, as illustrated in FIGURE 8b, the shape of an electrode 294' may be such that the metallic material in the region not intended to contact tissue is eliminated.

The masking techniques described in the preceding paragraphs improve the efficiency of, for example, an ablation procedure by decreasing the surface area of the electrodes and, therefore, the energy required to heat tissue. The masking can be used to form a narrow electrode which is sometimes desirable, even when the patient will be on bypass. The convective cooling effects of blood flowing by the electrode are also reduced. In addition, the transmission of RF energy to unintended anatomic structures is prevented. This is especially important in epicardial applications when the ablation electrode elements may be sandwiched between multiple anatomic structures including, for example, the aorta and pulmonary artery. The masking techniques also focus the application of ablating energy to helps to control the characteristics of the lesion.

## III.      Epicardial Applications of Probe-Type Apparatus

The embodiments of the present invention described above (primarily those discussed above with reference to FIGURES 10a-14) may be used in a variety of epicardial procedures. One such procedure is a maze-like ablation procedure to prevent atrial fibrillation. A thoracostomy, which is a surgical procedure that is less invasive than a thoracotomy or median sternotomy, may be used to gain access to the atrium. Here, relatively small incisions are created in the intercostal space. At each of the incisions, a trocar may be used to provide a port to access the thoracic cavity. These ports may be used for visualization with fiberoptic cameras, ultrasound, or other visualization devices, as well as for the surgical devices that ablate tissue. The surgical devices may be, for example, inserted through the ports located on the left side of the patient which provide direct access to the left atrium. The devices may then be used to create long, thin, curvilinear lesions or annular lesions on the epicardial surface. If necessary, lung lobes may be deflated during the procedure by inserting an endotracheal tube that inflates the right lung only. The left lung will collapse when the chest is opened.

There is also a high prevalence of atrial fibrillation substrates proximate to the pulmonary veins. Lesions may be created on the epicardial surface around pulmonary veins or between pulmonary veins. There is, however, some difficulty associated with epicardial access due to the presence of fatty deposits in the pulmonary vein region. The devices described above can create lesions on the epicardial surface proximate to the pulmonary veins because they can penetrate through fatty deposits and exert enough force against the epicardial surface to compress the remaining fat to such an extent that the ablation electrodes contact the epicardium. It is, however, very difficult to achieve suitable contact between the tissue and the electrodes. Thus, it is preferable to perform endocardial ablation around or between pulmonary veins in the manner described below.

## IV.      Endocardial Applications of Probe-Type Apparatus

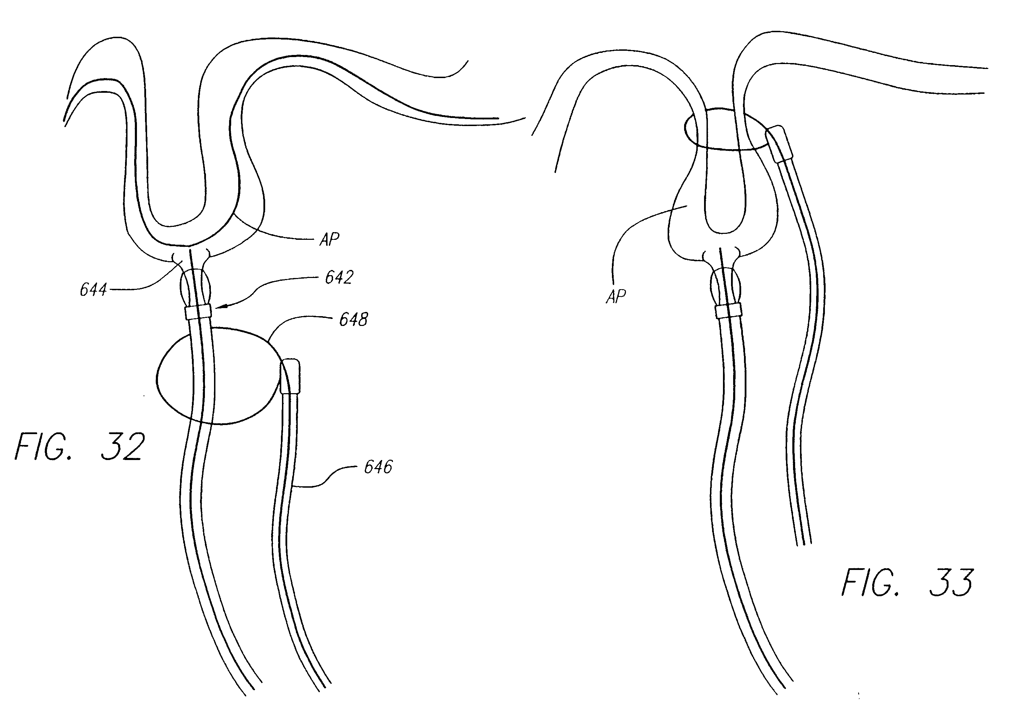

The embodiments of the present invention described above may be used in a variety of endocardial procedures. To create lesions on the endocardial surface, access to the interior of the left atrium must also be obtained. To obtain thoracoscopic access to the left atrium via a thoracostomy, a cannula may be inserted through the left atrial appendage or the left atrial free wall. The preferred access point is the left atrial appendage, especially if the physician intends to isolate the left atrial appendage at the end of the procedure. More specifically, and as shown by way of example in FIGURES 32 and 33, a grabbing catheter 642 having movable grasping prongs 644 may be used to capture, pull and stretch the appendage AP. Next, a lasso catheter 646 having a lasso 648 may be used to encircle the left atrial appendage near the base of the appendage. The grabbing catheter facilitates the positioning of the lasso at the base of the appendage by pulling the appendage through the lasso. A needle is then used to puncture the appendage wall and gain access to the left atrium. A guidewire is advanced through the needle into the left atrium. The needle is then removed, leaving the guidewire in place. An introducer/dilator combination is then advanced over the guidewire into the left atrium. Next, the lasso is then tightened around the introducer to prevent blood flow past the introducer into the distal region of the atrial appendage. The dilator is then removed, leaving the introducer as the access to the interior of the left atrium.

Instead of the lasso technique, a purse string technique may be employed wherein sutures are used to tighten the atrial appendage around the introducer.

One of the exemplary devices described above, such as those described with reference to FIGURES 1-9f, may then be inserted into the atrium with its spline collapsed. Once inside, the sheath is retracted such that the spline returns to its predetermined configuration and the ablation procedure is performed. The sheath is pushed over the spline when the ablation procedure is complete and the device is removed from the atrium. Similarly, the devices described above with reference to FIGURES 14 and 15 may be inserted with the loop in its retracted state, while the device shown in FIGURE 13 may be inserted prior to pulling the wire attached to the distal tip. These devices may then be manipulated to cause the loops to form. The ablation procedure can then be performed. The devices described above with reference to FIGURES 10a-c, 12 and 16 need only be inserted to perform the procedure. The same is also true for malleable versions of the exemplary devices shown in FIGURES 1-9e.

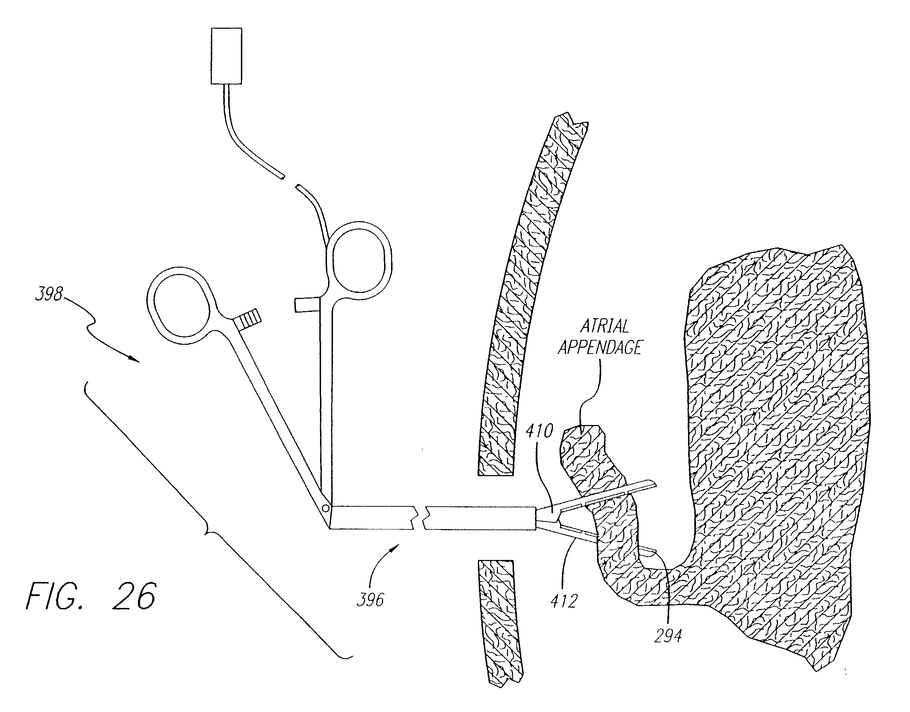

Upon completion, the introducer is removed and the lasso tightened to isolate the left atrial appendage. The lasso may be detached from the probe and left in place to keep the appendage isolated. Where the aforementioned purse string technique is employed, the sutures may be tightened isolate the appendage. Alternatively, the appendage may be isolated in the manner described below with reference to FIGURE 26.

In addition to thoracoscopic procedures, another area of cardiac treatment which will benefit from the present invention is the repair and replacement of mitral valves (which typically involves a thoracotomy, median sternotomy, or thoracostomy) because atrial fibrillation can be a complication of mitral disease which occurs prior to or subsequent to mitral valve surgery. More specifically, incisional reentry can develop subsequent to surgical procedures (such as mitral valve and thoracoscopic procedures) where an incision is made in the atrial wall that is subsequently closed by either sutures, mechanical closures, or other similar devices. Creating a lesion from the incision to the mitral valve annulus (or other anatomic barrier) will reduce the potential for reentrant propagation around the incision and, therefore, will terminate atrial fibrillation and/or prevent atrial fibrillation from developing. For example, if the left atrial appendage is used to access the interior of the left atrium for devices that create lesions on the endocardial surface, an additional lesion should be created from this access site to the mitral valve annulus so that incisional reentry will not develop when the incision is closed. This additional procedure is also applicable for right atrial procedures using incisions to access the interior of the atrium.

There is also a high prevalence of atrial fibrillation substrates proximate to the pulmonary veins. The creation of long, curvilinear lesions between pulmonary veins, around single pulmonary veins, and/or from pulmonary veins to the mitral valve annulus will prevent atrial fibrillation. The exemplary device illustrated FIGURES 1 and 2, which has an annular electrode assembly, is especially well suited for positioning ablation electrodes around the inside of a pulmonary vein. Alternatively, lesions may be created on the epicardial surface around pulmonary veins or between pulmonary veins. There is, however, some difficulty associated with epicardial access due to the presence of fatty deposits in the pulmonary vein region.

## V. Other Surgical Applications

A surgical procedure employing the present invention may be used to reduce the level of bleeding during surgical procedures. The procedure generally comprises the steps of coagulating (or ablating) tissue to a predetermined depth and then forming an incision in the coagulated tissue. The coagulation can be accomplished by applying RF energy with, for example, the probe shown in FIGURE 10a. Because the tissue is coagulated, the incision will not result in bleeding.

One exemplary procedure is the removal of a diseased liver lobe. This a relatively time consuming procedure and, using conventional surgical techniques, there is a significant risk of serious bleeding. In using one embodiment of the present invention, tissue in the lobe is coagulated to a depth of approximately 3 mm to 7 mm using RF energy. The coagulated tissue is then cut and separated with a scalpel, electro-surgical device, or other suitable instrument. To avoid bleeding, the depth of the cut should not exceed the depth of the coagulated tissue. The process of coagulating tissue and then forming an incision in the coagulated tissue can be repeated until the incision reaches the desired depth. Here, each coagulation and incision cycle will take approximately 90 seconds, 60 seconds to perform the coagulation and 30 seconds to perform the incision.

The present surgical technique is, of course, applicable to surgical procedures in addition to the removal of a liver lobe. Such procedures may, for example, involve the spleen, the kidneys, other areas of the liver, the heart, skeletal muscle, the lungs (such as a pulmonary lobotomy) and the brain. The present technique is also useful in oncological surgical procedures because cancerous tumors tend to be highly vascularized. One exemplary oncological procedure is the debulking of a cancerous tumor.

A surgical tool set in accordance with a present invention includes, among the other tools needed for a particular procedure, a device for coagulating soft tissue and a cutting the tissue. Suitable devices for coagulating soft tissue are illustrated for example, in FIGURES 1-27 and 34-40. With respect to the probe shown in FIGURES 10f and 10g, the portion of the probe which includes the second connector portion 321, the shaft 310 and a plurality of electrode elements can be included in the tool set with or without the handle 312". As noted above, scalpels, electro-surgical devices and other suitable instruments may be used to cut tissue. Preferably, the tool set is housed in a sterile package that has a flat rigid bottom portion and a top transparent top cover that provides recesses for the tools, thereby providing a ready to use surgical kit. The bottom portion may be formed from Tyvek® spun bonded plastic fibers, or other suitable materials, which allow the contents of the package to be sterilized after the tools are sealed within the package.

## VI. Apparatus that Apply a Clamping Force

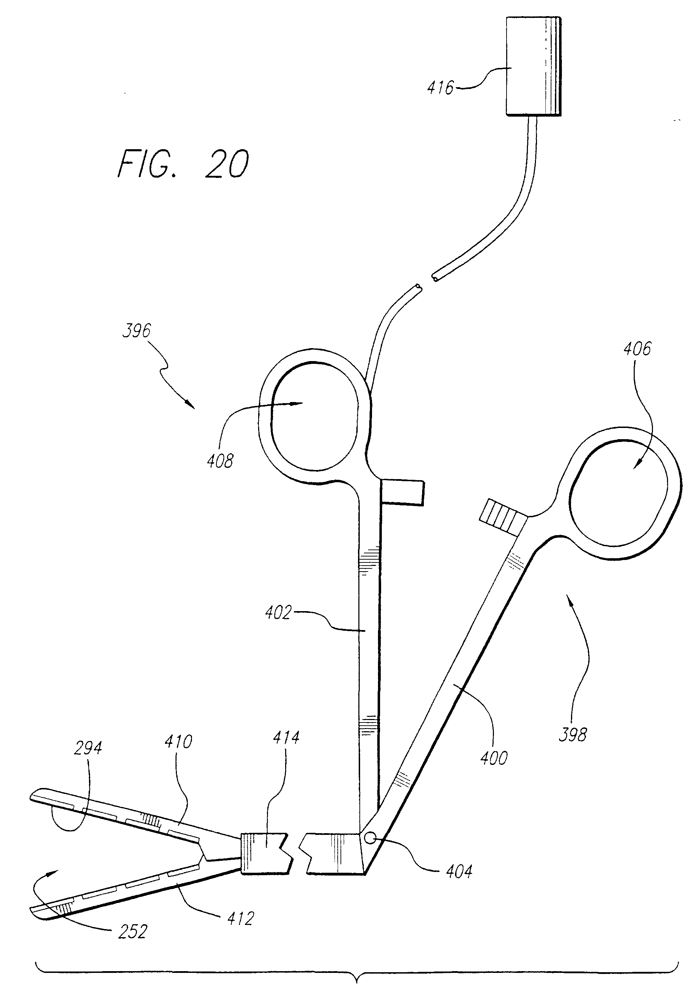

In accordance with another embodiment of the present invention, and as shown by way of example in FIGURES 17-19, a clamp 382 includes a pair of clamp members 384 and 386, which are pivotally secured to one another by a pin 388, and an operative element 252 that may be of the type discussed above in Section II. Here, the operative element consists of a plurality of ablation electrodes 294. The clamp 382 also includes a pair of locking members 390 and 392 and an electrical connector 394 that may be used to, for example, connect the electrodes 294 to a source RF energy. Referring more specifically to FIGURE 19, the clamp 382 may also, if desired, be curved over its length. Of course, the overall shape of the clamp will depend upon the procedure for which it is intended.

Certain procedures require the application of a clamping force to the bodily structure of interest in addition to the operation performed by the operative element. One such procedure is the isolation of an atrial appendage, which is discussed in greater detail below with reference to FIGURE 26. As illustrated for example in FIGURE 20, a suitable surgical device 396 for use in such a procedure includes a handle 398 having a pair of handle members 400 and 402 which are movable relative to one another. In the exemplary embodiment, the handle members are pivotably secured to one another by a pin 404 and include respective openings 406 and 408. The handle 398, which is actuated in a manner similar to scissors, is operably connected to a pair of support members 410 and 412 by, for example, a suitable mechanical linkage located within a housing 414. Actuation of the handle 398 causes the support members 410 and 412 to move relative to one another to create a clamping force. Of course, other types of handles that can cause movement of the support members may also be used.

An operative element 252, is associated with one or both (as shown) of the support members 410 and 412. Preferably, the operative element consists of one or more electrode elements 294 suitable for ablation (such as those discussed in detail in Section II above and operable in either the uni-polar or bi-polar mode) on each of the support members 410 and 412. Of course, the operative element 252 may also consist in whole or in part of other types of electrodes, such as a hot tip to cauterize appendage walls. The electrode elements 294 (or other operative element) may be connected to a control/power source surgical device by way of a connector 416. Wires extend from the electrode elements 294 through lumens in the support members 410 and 412 and handle 398 to the connector 416.

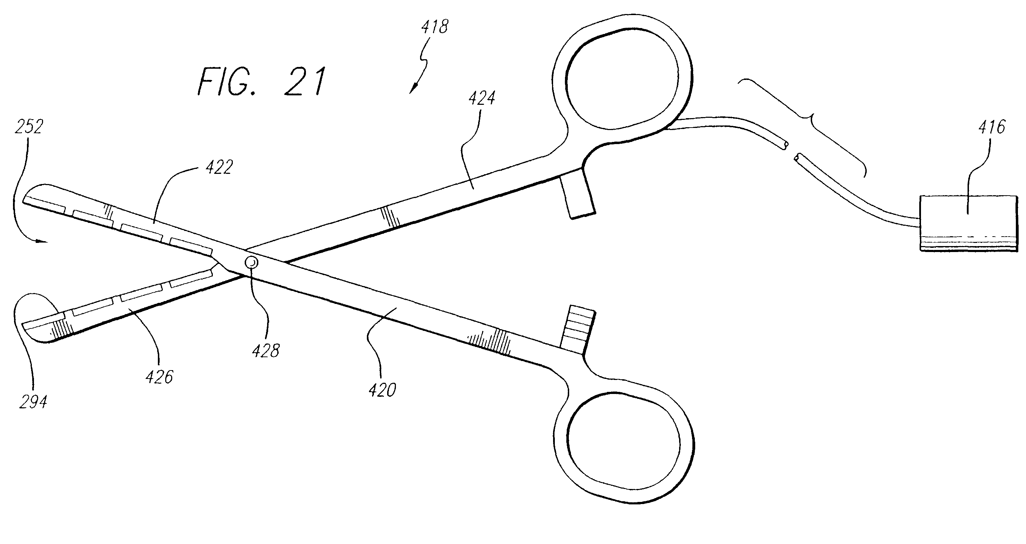

Turning to FIGURE 21, surgical device 418 is similar to that shown in FIGURE 20 except that handle 398 is not connected to the operative element support members 410 and 412 by a mechanical linkage. Instead, the handle member 420 and support member 422 form an integral unit as do the handle member 424 and support member 426. The integral units are pivotably secured to one another by a pin 428. Thus, while the embodiment shown in FIGURE 20 is especially useful in situations where thoracostomy is used, the embodiment shown in FIGURE 21 is especially useful for thoracotomy or median sternotomy access. In either case, the atrial appendage (or other bodily structure) is captured (or clamped) such that it is perpendicular to the surgical device.

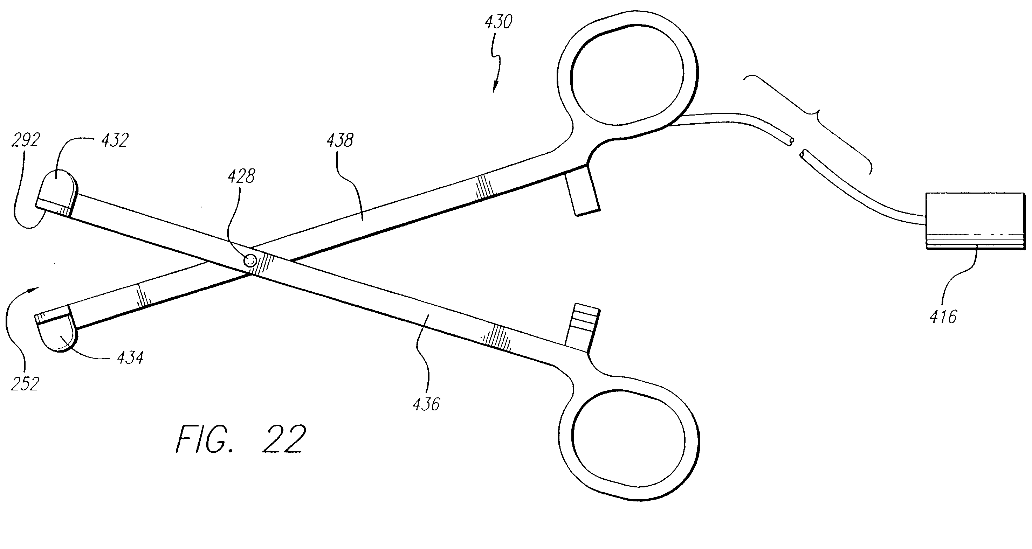

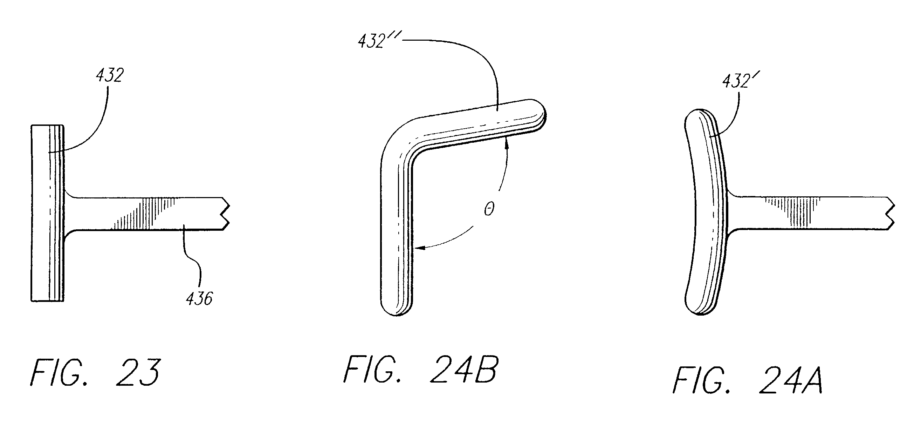

As shown by way of example in FIGURES 22 and 23, the operative element support members 432 and 434 in exemplary surgical device 430 are secured to the distal ends of the handle members 436 and 438, respectively, such that the support members are perpendicular to the handle members. Although the handle members 436 and 438 are respectively secured to the middle portion of the support members 432 and 434 (viewed longitudinally as shown in FIGURE 23), the support members may be offset in one direction or the other to suit particular needs (note FIGURE 23a). Additionally, as illustrated for example in FIGURES 24a and 24b, the support members (432' and 432") may also be curved, or L-shaped with the angle θ between about 90° and about 180°. The preferred embodiments shown in FIGURES 22-24b hold the bodily structure such that it is parallel to the surgical device.

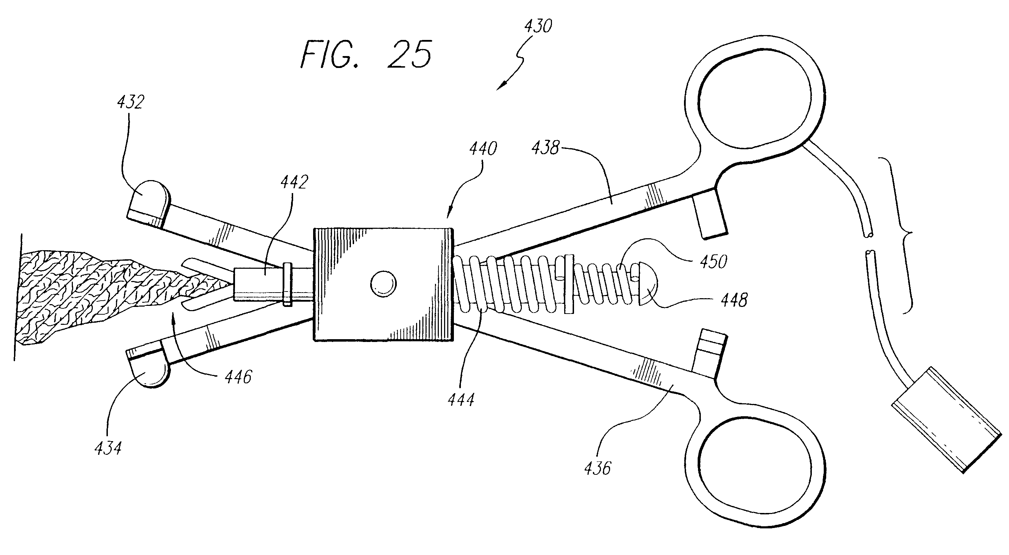

The exemplary embodiments shown in FIGURES 22-24b may be provided with a holding device that is used to grasp a bodily structure and pull the structure in the proximal direction. As illustrated for example in FIGURE 25, the holding device 440 includes a cylindrical member 442 that is biased in the proximal direction by a spring 444. A pair of clamping jaws 446 extend outwardly from the distal end of the cylindrical member 442. The clamping jaws 446, which pivot relative to one another, are connected to a rod 448 which passes through the cylindrical member 442 and slides relative thereto. The rod 448 is biased in the proximal direction by a spring 450 which, in turn, biases the clamping jaws 446 in the proximal direction against the distal end of the cylindrical member 442. As such, the clamping jaws 446 are biased to their closed position and the jaws may be loosened by pushing the rod 448 in the distal direction.

## VII. Applications of Apparatus that Apply a Clamping Force

The exemplary clamp 382 shown in FIGURES 17-19 can both isolate a bodily structure and deliver the therapeutic and/or diagnostic effects of the operative element 252. In an atrial appendage isolation procedure, for example, the clamp 382 may be used to capture the atrial appendage and isolate it from the interior of the atrium. RF energy may then be delivered via the electrodes 294 (in either the uni-polar mode or the bi-polar mode) to fuse the walls of the atrial appendage to one another. Thereafter, the clamp may either be removed, or disconnected from the RF energy source and left in place.

Turning to FIGURE 26, one exemplary use of the surgical device 396 shown in FIGURE 20 is the isolation of an atrial appendage. Here, the device is inserted into an opening of the chest wall. The atrial appendage is captured between the support members 410 and 412 by actuating the handle 398. RF energy is then transmitted, either from the electrodes 294 on one support member to the electrodes on the other (bi-polar mode) or from the electrodes to an indifferent reference electrode on, for example, a patch (uni-polar mode) to thermally fuse the walls of the atrial appendage together and isolate the atrial appendage. The surgical device shown in FIGURES 21-26 may be used in similar fashion.

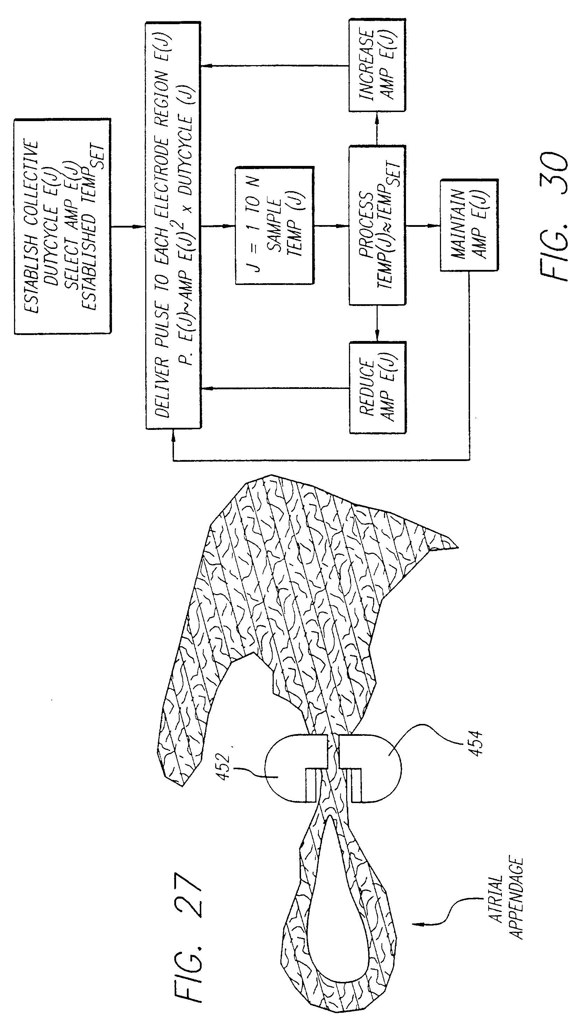

As shown by way of example in FIGURE 27, the operative element (such as, for example, electrodes 294) may be offset from one side or the other of the support members 452 and 454. This offset configuration, which may be used in conjunction with any of the exemplary devices shown in FIGURES 20-25, is especially useful in an atrial appendage isolation procedure. Here, the electrodes 294 are offset from the side of the support members 452 and 454 that is proximate to the interior of the left atrium. By making the portions of the support members that do not support the electrodes insulative, and by directing the RF energy towards the side of the appendage (or other structure) isolated by the clamping force, coagulum or thrombus due to heating static blood will develop in the portion of the appendage that will be isolated from the blood pool when the side walls fuse to one another. Of course, when the patient is in bypass, such masking is unnecessary unless it is being used to create lesions of a certain shape.

## VIII. Power Control

### A. General

FIGURE 28 shows, in schematic form, a representative system 500 for applying ablating energy by multiple emitters based, at least in part, upon local temperature conditions sensed by multiple sensing elements.

In FIGURE 28, the multiple sensing elements comprise thermocouples 508, 509, and 510 individually associated with the multiple emitters of ablating energy, which comprise electrode regions 501, 502, and 503. The system 500 also includes a common reference thermocouple 511 carried within the coupler element for exposure to the blood pool. Alternatively, other kinds of temperature sensing elements can be used, like, for example, thermistors, fluoroptic sensors, and resistive temperature sensors, in which case the reference thermocouple 511 would typically not be required.

The system 500 further includes an indifferent electrode 519 for operation in a uni-polar mode.

The ablating energy emitters 501, 502, 503 can comprise the rigid electrode segments previously described. Alternatively, the electrode regions 501, 502, 503 can comprise a continuous or segmented flexible electrode of wrapped wire or ribbon. It should be appreciated that the system 500 can be used in association with any ablating element that employs multiple, independently actuated ablating elements.

The system 500 includes a source 517 of ablating energy. In FIGURE 28, the source 517 generates radio frequency (RF) energy. The source 517 is connected (through a conventional isolated output stage 516) to an array of power switches 514, one for each electrode region 501, 502, and 503. A connector 512 (carried by the probe handle) electrically couples each electrode region 501, 503, 503 to its own power switch 514 and to other parts of the system 500.

The system 500 also includes a microcontroller 531 coupled via an interface 530 to each power switch 514. The microcontroller 531 turns a given power switch 514 on or off to deliver RF power from the source 517 individually to the electrode regions 501, 502, and 503. The delivered RF energy flows from the respective electrode region 501, 502, and 503, through tissue, to the indifferent electrode 519, which is connected to the return path of the isolated output stage 516.

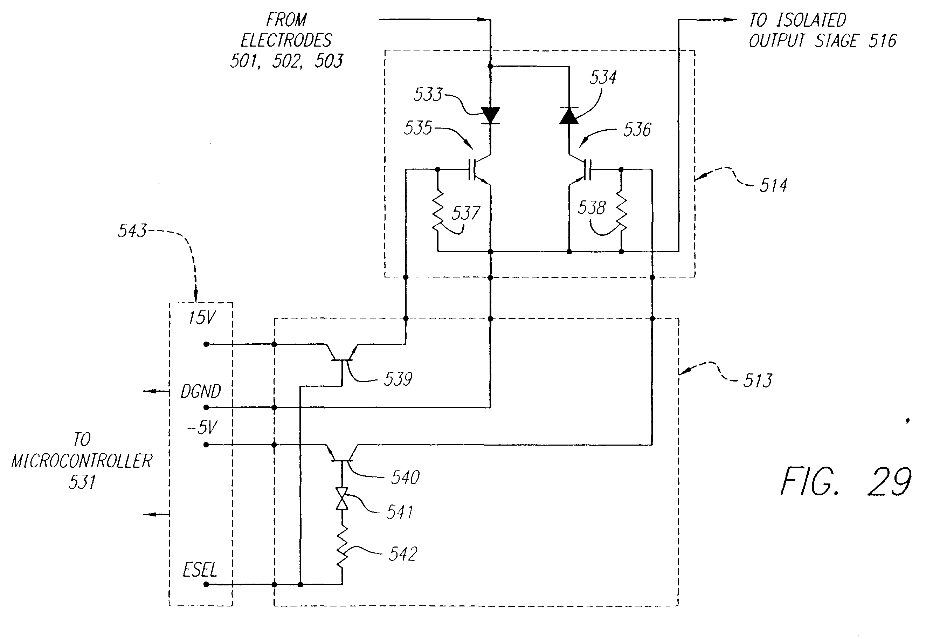

The power switch 514 and interface 530 configuration can vary according to the type of ablating energy being applied. FIGURE 29 shows a representative implementation for applying RF ablating energy.

In this implementation, each power switch 514 includes an N-MOS power transistor 535 and a P-MOS power transistor 536 coupled in between the respective electrode region 501, 502, and 503 and the isolated output stage 516 of the power source 517.

A diode 533 conveys the positive phase of RF ablating energy to the electrode region. A diode 534 conveys the negative phase of the RF ablating energy to the electrode region. Resistors 537 and 538 bias the N-MOS and P-MOS power transistors 535 and 536 in conventional fashion.

The interface 530 for each power switch 514 includes two NPN transistors 539 and 540. The emitter of the NPN transistor 539 is coupled to the gate of the N-MOS power transistor 535. The collector of the NPN transistor 540 is coupled to the gate of the P-MOS power transistor 534,

The interface for each power switch 514 also includes a control bus 543 coupled to the microcontroller 531. The control bus 543 connects each power switch 514 to digital ground (DGND) of the microcontroller 531. The control bus 543 also includes a (+) power line (+5V) connected to the collector of the NPN transistor 539 and a (-) power line (-5V) connected to the emitter of the NPN interface transistor 540.

The control bus 543 for each power switch 514 further includes an $E_{SEL}$ line. The base of the NPN transistor 539 is coupled to the $E_{SEL}$ line of the control bus 543. The base of the NPN transistor 540 is also coupled to the $E_{SEL}$ line of the control bus 543 via the Zener diode 541 and a resistor 532. The $E_{SEL}$ line connects to the cathode of the Zener diode 541 through the resistor 532. The Zener diode 541 is selected so that the NPN transistor 540 turns on when $E_{SEL}$ exceeds about 3 volts (which, for the particular embodiment shown, is logic 1).

It should be appreciated that the interface 530 can be designed to handle other logic level standards. In the particular embodiment, it is designed to handle conventional TTL (transistor transfer logic) levels.

The microcontroller 531 sets $E_{SEL}$ of the control bus 543 either at logic 1 or at logic 0. At logic 1, the gate of the N-MOS transistor 535 is connected to (+) 5 volt line through the NPN transistors 539. Similarly, the gate of the P-MOS transistor 536 is connected to the (-) 5 volt line through the NPN transistor 540. This conditions the power transistors 535 and 536 to conduct RF voltage from the source 517 to the associated electrode region. The power switch 514 is "on."

When the microcontroller 531 sets $E_{SEL}$ at logic 0, no current flows through the NPN transistors 539 and 540. This conditions the power transistors 535 and 536 to block the conduction of RF voltage to the associated electrode region. The power switch 514 is "off."

The system 500 (see FIGURE 28) further includes two analog multiplexers (MUX) 524 and 525. The multiplexers 524 and 525 receive voltage input from each thermocouple 508, 509, 510, and 511. The microcontroller 531 controls both multiplexers 524 and 525 to select voltage inputs from the multiple temperature sensing thermocouples 508, 509, 510, and 511.

The voltage inputs from the thermocouples 508, 509, 510, and 511 are sent to front end signal conditioning electronics. The inputs are amplified by differential amplifier 526, which reads the voltage differences between the copper wires of the thermocouples 508/509/510 and the reference thermocouple 511. The voltage differences are conditioned by element 527 and converted to digital codes by the analog-to-digital converter 528. The look-up table 529 converts the digital codes to temperature codes. The temperature codes are read by the microcontroller 531.

The microcontroller 531 compares the temperature codes for each thermocouple 508, 509, and 510 to preselected criteria to generate feedback signals. The preselected criteria are inputted through a user interface 532. These feedback signals control the interface power switches 514 via the interface 530, turning the electrodes 501, 502, and 503 off and on.

The other multiplexer 525 connects the thermocouples 508, 509, 510, and 511 selected by the microcontroller 531 to a temperature controller 515. The temperature controller 515 also includes front end signal conditioning electronics, as already described with reference to elements 526, 527, 528, and 529. These electronics convert the voltage differences between the copper wires of the thermocouples 508/509/510 and the reference thermocouple 511 to temperature codes. The temperature codes are read by the controller and compared to preselected criteria to generate feedback signals. These feedback signals control the amplitude of the voltage (or current) generated by the source 517 for delivery to the electrodes 501, 502, and 503.

Based upon the feedback signals of the microcontroller 531 and the temperature controller 515, the system 500 distributes power to the multiple electrode regions 501, 502, and 503 to establish and maintain a uniform distribution of temperatures along the ablating element. In this way, the system 500 obtains safe and efficacious lesion formation using multiple emitters of ablating energy.

The system 500 can control the delivery of ablating energy in different ways. Representative modes will now be described.

**B.    Individual Amplitudes/Collective Duty Cycle**

The electrode regions 501, 502, and 503 will be symbolically designated E(J), where J represents a given electrode region (J = 1 to N).

As before described, each electrode region E(J) has at least one temperature sensing element 508, 509, and 510, which will be designated S(J,K), where J represents the electrode region and K represents the number of temperature sensing elements on each electrode region (K = 1 to M).

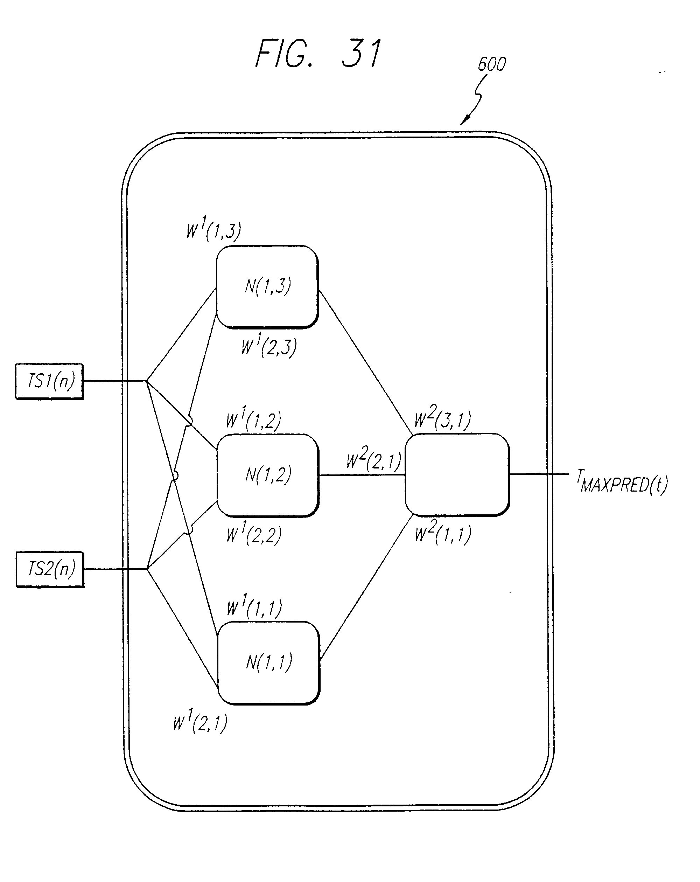

In this mode (see FIGURE 30), the microcontroller 516 operates the power switch interface 530 to deliver RF power from the source 517 in multiple pulses of duty cycle 1/N.

With pulsed power delivery, the amount of power ($P_{E(J)}$) conveyed to each individual electrode is as follows:

$$P_{E(J)} \sim AMP_{E(J)}{}^2 \times DUTYCYCLE_{E(J)}$$

where:

$AMP_{E(J)}$ is the amplitude of the RF voltage conveyed to the electrode region E(J), and
$DUTYCYCLE_{E(J)}$ is the duty cycle of the pulse, expressed as follows:

$$DUTYCYCLE_{E(J)} = TON_{E(J)}/[TON_{E(J)} + TOFF_{E(J)}]$$

where:

$TON_{E(J)}$ is the time that the electrode region E(J) emits energy during each pulse period,
$TOFF_{E(J)}$ is the time that the electrode region E(J) does not emit energy during each pulse period.

The expression $TON_{E(J)} + TOFF_{E(J)}$ represents the period of the pulse for each electrode region E(J).

In this mode, the microcontroller 531 collectively establishes duty cycle ($DUTYCYCLE_{E(J)}$) of 1/N for each electrode region (N being equal to the number of electrode regions).

The microcontroller 531 may sequence successive power pulses to adjacent electrode regions so that the end of the duty cycle for the preceding pulse overlaps slightly with the beginning of the duty cycle for the next pulse. This overlap in pulse duty cycles assures that the source 517 applies power continuously, with no periods of interruption caused by open circuits during pulse switching between successive electrode regions.

In this mode, the temperature controller 515 makes individual adjustments to the amplitude of the RF voltage for each electrode region ($AMP_{E(J)}$), thereby individually changing the power $P_{E(J)}$ of ablating energy conveyed during the duty cycle to each electrode region, as controlled by the microcontroller 531.

In this mode, the microcontroller 531 cycles in successive data acquisition sample periods. During each sample period, the microcontroller 531 selects individual sensors S(J,K), and voltage differences are read by the controller 515 (through MUX 525) and converted to temperature codes TEMP(J).

When there is more than one sensing element associated with a given electrode region, the controller 515 registers all sensed temperatures for the given electrode region and selects among these the highest sensed temperature, which constitutes TEMP(J).

In this mode, the controller 515 compares the temperature TEMP(J) locally sensed at each electrode E(J) during each data acquisition period to a set point temperature $TEMP_{SET}$ established by the physician. Based upon this comparison, the controller 515 varies the amplitude $AMP_{E(J)}$ of the RF voltage delivered to the electrode region E(J), while the microcontroller 531 maintains the $DUTYCYCLE_{E(J)}$ for that electrode region and all other electrode regions, to establish and maintain TEMP(J) at the set point temperature $TEMP_{SET}$.

The set point temperature $TEMP_{SET}$ can vary according to the judgment of the physician and empirical data. A representative set point temperature for cardiac ablation is believed to lie in the range of 40° C to 95° C, with 70° C being a representative preferred value.

The manner in which the controller 515 governs $AMP_{E(J)}$ can incorporate proportional control methods, proportional integral derivative (PID) control methods, or fuzzy logic control methods.

For example, using proportional control methods, if the temperature sensed by the first sensing element TEMP(1) > $TEMP_{SET}$, the control signal generated by the controller 515 individually reduces the amplitude $AMP_{E(1)}$ of the RF voltage applied to the first electrode region E(1), while the microcontroller 531 keeps the collective duty cycle $DUTYCYCLE_{E(1)}$ for the first electrode region E(1) the same. If the temperature sensed by the second sensing element TEMP(2) < $TEMP_{SET}$, the control signal of the controller 515 increases the amplitude $AMP_{E(2)}$ of the pulse applied to the second electrode region E(2), while the microcontroller 531 keeps the collective duty cycle $DUTYCYCLE_{E(2)}$ for the second electrode region E(2) the same as $DUTYCYCLE_{E(1)}$, and so on. if the temperature sensed by a given sensing element is at the set point temperature $TEMP_{SET}$, no change in RF voltage amplitude is made for the associated electrode region.

The controller 515 continuously processes voltage difference inputs during successive data acquisition periods to individually adjust $AMP_{E(J)}$ at each electrode region E(J), while the microcontroller 531 keeps the collective duty cycle the same for all electrode regions E(J). In this way, the mode maintains a desired uniformity of temperature along the length of the ablating element.

Using a proportional integral differential (PID) control technique, the controller 515 takes into account not only instantaneous changes that occur in a given sample period, but also changes that have occurred in previous sample periods and the rate at which these changes are varying over time. Thus, using a PID control technique, the controller 515 will respond differently to a given proportionally large instantaneous difference between TEMP (J) and $TEMP_{SET}$, depending upon whether the difference is getting larger or smaller, compared to previous instantaneous differences, and whether the rate at which the difference is changing since previous sample periods is increasing or decreasing.

## C. Deriving Predicted Hottest Temperature

Because of the heat exchange between the tissue and the electrode region, the temperature sensing elements may not measure exactly the maximum temperature at the region. This is because the region of hottest temperature occurs beneath the surface of the tissue at a depth of about 0.5 to 2.0 mm from where the energy emitting electrode region (and the associated sensing element) contacts the tissue. If the power is applied to heat the tissue too quickly, the actual maximum tissue temperature in this subsurface region may exceed 100° C and lead to tissue desiccation and/or micro-explosion.

FIGURE 31 shows an implementation of a neural network predictor 600, which receives as input the temperatures sensed by multiple sensing elements S(J,K) at each electrode region, where J represents a given electrode region (J = 1 to N) and K represents the number of temperature sensing elements on each electrode region (K = 1 to M). The predictor 600 outputs a predicted temperature of the hottest tissue region $T_{MAXPRED}(t)$. The controller 515 and microcontroller 531 derive the amplitude and duty cycle control signals based upon $T_{MAXPRED}(t)$, in the same manners already described using TEMP(J).

The predictor 600 uses a two-layer neural network, although more hidden layers could be used. As shown in FIGURE 30, the predictor 600 includes first and second hidden layers and four neurons, designated $N_{(L,X)}$, where L identifies the layer 1 or 2 and X identifies a neuron on that layer. The first layer (L=1) has three neurons (X = 1 to 3), as follows $N_{(1,1)}$; $N_{(1,2)}$; and $N_{(1,3)}$. The second layer (L=2) comprising one output neuron (X=1), designated $N_{(2,1)}$.

Temperature readings from the multiple sensing elements, only two of which -- TS1(n) and TS2(n) -- are shown for purposes of illustration, are weighed and inputted to each neuron $N_{(1,1)}$; $N_{(1,2)}$; and $N_{(1,3)}$ of the first layer. FIGURE 30 represents the weights as $W^L_{(k,N)}$, where L=1; k is the input sensor order; and N is the input neuron number 1, 2, or 3 of the first layer.

The output neuron $N_{(2,1)}$ of the second layer receives as inputs the weighted outputs of the neurons $N_{(1,1)}$; $N_{(1,2)}$; and $N_{(1,3)}$. FIGURE 30 represents the output weights as $W^L_{(O,X)}$, where L=2; O is the output neuron 1, 2, or 3 of the first layer; and X is the input neuron number of the second layer. Based upon these weighted inputs, the output neuron $N_{(2,1)}$ predicts $T_{MAXPRED}(t)$. Alternatively, a sequence of past reading samples from each sensor could be used as input. By doing this, a history term would contribute to the prediction of the hottest tissue temperature.

The predictor 600 must be trained on a known set of data containing the temperature of the sensing elements TS1 and TS2 and the temperature of the hottest region, which have been previously acquired experimentally. For example, using a back-propagation model, the predictor 600 can be trained to predict the known hottest temperature of the data set with the least mean square error. Once the training phase is completed the predictor 600 can be used to predict $T_{MAXPRED}(t)$.

Other types of data processing techniques can also be used to derive $T_{MAXPRED}(t)$.

It should be noted that there are certain considerations which should be taken into account when ablation/coagulation procedures are performed with little or no fluid present. Such procedures include, for example, procedures performed during cardiac bypass. These considerations stem from the fact that the convective cooling effects associated with air are far less than that associated with blood and other fluids. In addition, the intimate physical (and thermal) contact between the electrodes and tissue will allow heat to be exchanged relatively freely therebetween.

Because the electrodes which transmit RF energy have high conductivity, they will be subjected to much less ohmic heating. However, heat will be drawn from the tissue to the electrode as RF power is applied to the tissue, which results in a time lag between hottest tissue temperature and the temperature of the electrode as well as a temperature gradient within the tissue near the tissue surface. The electrode temperature will eventually approach the tissue temperature. At this point, there will be a relatively small temperature gradient between the hottest tissue temperature and the electrode temperature, as well as relatively little heat transfer between the tissue and the electrode. Accordingly, the temperature control algorithm should take into account the time lag between the sub-surface tissue temperature and the temperature sensed at the electrode. However, the difference between the plateau tissue temperatures and the sensed temperatures can typically be disregarded.

In addition to the control considerations, the user interface should also allow the physician to indicated whether convective cooling is going to be present, thereby allowing the physician to select the proper temperature control algorithm.

The illustrated and preferred embodiments used digital processing controlled by a computer to analyze information and generate feedback signals. It should be appreciated that other logic control circuits using microswitches, AND/OR gates, invertors, analog circuits, and the like are equivalent to the micro-processor controlled techniques shown in the preferred embodiment.

Although the present invention has been described in terms of the preferred embodiments above, numerous modifications and/or additions to the above-described preferred embodiments would be readily apparent to one skilled in the art. It is intended that the scope of the present invention extends to all such modifications and/or additions.

**Claims**

1.  A soft tissue coagulation probe (308) comprising a handle (312) and at least two spaced soft tissue coagulation electrodes (294) supported on an electrode support assembly located in spaced relation to the handle, the respective size and spacing of the at least two soft tissue coagulation electrodes (294) being such that simultaneous transmission of energy thereby to an indifferent electrode will produce an area of coagulated tissue that spans the at least two soft tissue coagulation electrodes, **characterized by**
    a relatively short malleable shaft (310) having a proximal end associated with the handle (312) and a distal end connected to the electrode support assembly, said malleable shaft having a bending modulus of between approximately 3 lb.-in.$^2$ (86 N-cm$^2$) and approximately 50 lb.-in.$^2$ (1435 N-cm$^2$), wherein the bending modulus is the product of the modulus of elasticity and of the moment of inertia of the shaft (310).

2.  A soft tissue coagulation probe as claimed in claim 1, further comprising:

    a temperature sensor located at the longitudinal end edge of at least one of the soft tissue coagulation electrodes.

3.  A soft tissue coagulation probe as claimed in claim 1, further comprising:

    a temperature sensor associated with at least one of the soft tissue coagulation electrodes.

4.  A soft tissue coagulation probe as claimed in claim 1, wherein the electrode support assembly comprises a bendable spline assembly (256) at the distal end thereof having a predetermined configuration, the spline assembly being adapted to collapse in response to an application of an external force and to expand to the predetermined configuration in response to a withdrawal of the external force, the soft tissue coagulation electrodes (294) being located on the bendable spline assembly.

5. A soft tissue coagulation probe as claimed in claim 4, further comprising:

a substantially tubular member (264) positioned around the shaft (254) and slidable relative to the shaft;

wherein the spline assembly (256) is adapted to collapse in response to movement of the substantially tubular member thereover and to expand to the predetermined configuration in response to a retraction of the substantially tubular member.

6. A soft tissue coagulation probe as claimed in claim 4, wherein the bendable spline assembly (256) includes a substantially annular portion (262).

7. A soft tissue coagulation probe as claimed in claim 6, wherein the shaft (254) defines a longitudinal axis and the substantially annular portion (262) lies in a plane oriented substantially perpendicular to the longitudinal axis.

8. A soft tissue coagulation probe as claimed in claim 4, wherein the bendable spline assembly comprises a substantially triangularly-shaped spline assembly (272).

9. A soft tissue coagulation probe as claimed in claim 8, wherein the substantially triangularly-shaped spline assembly (272) comprises a distal leg (278) defining first and second ends, the distal leg being non-linear from the first end to the second end.

10. A soft tissue coagulation probe as claimed in claim 6, wherein the bendable spline assembly is in the form of a loop (300).

11. A soft tissue coagulation probe as claimed in claim 1, wherein the electrode support assembly comprises a distal tip assembly (350) including a distal member that is one of flexible, somewhat flexible and malleable, the soft tissue coagulation electrodes (294) being located on the distal member.

12. A soft tissue coagulation probe as claimed in claim 11, further comprising:

a tubular member (354) positioned around a predetermined portion of the shaft and movable relative thereto, the tubular member defining a proximal end, a distal end and an opening between the distal and proximal ends;

wherein the distal tip assembly includes a pivot assembly (360) having a first pivot member (364) connected to a distal portion of the distal member and a second pivot member (362) connected to the tubular member.

13. A soft tissue coagulation probe as claimed in claim 1, wherein the handle (312") includes an energy control device (315) adapted to selectively control the transmission of energy from an energy source to the at least two soft tissue coagulation electrodes (294).

14. A soft tissue coagulation probe as claimed in claim 13, wherein the energy control device (315) comprises an on-off switch.

15. A soft tissue coagulation probe as claimed in claim 13, wherein the energy control device (315) comprises at least two energy control devices respectively coupled to the at least two soft tissue coagulation electrodes (294).

16. A soft tissue coagulation probe as claimed in claim 15, further comprising:

at least two indicators (317) adapted to indicate the respective statuses of the at least two soft tissue coagulation electrodes (294).

17. A soft tissue coagulation probe as claimed in claim 15, further comprising:

a global energy control device (313) adapted to selectively control the transmission of energy from the energy source to each of the soft tissue coagulation electrodes (294).

18. A soft tissue coagulation probe as claimed in claim 13, further comprising:

an indicator (317) adapted to indicate the status of the energy control device.

19. A soft tissue coagulation probe as claimed in claim 1, further comprising:

a connector (319) adapted to releasably connect the handle to the shaft.

20. A soft tissue coagulation probe as claimed in claim 1, further comprising:

a pressure application probe (650) including an elongate main body portion (652) and an engagement device (654) adapted to releasably engage the shaft.

21. A soft tissue coagulation probe as claimed in claim 20, wherein the engagement device (654) comprises a generally c-shaped member.

22. A soft tissue coagulation probe as claimed in claim 1, further comprising:

a coupling device (664) including a base member (666) adapted to be removably secured to a first portion of the shaft and an engagement device (668) connected to the base member and adapted to be removably secured to a second portion of the shaft;

wherein the shaft defines a loop when the first portion is secured to the base member and the second portion is secured to the coupling device.

**Patentansprüche**

1. Weichgewebe-Koagulationssonde (308), aufweisend einen Griff (312) und mindestens zwei in einem Abstand zueinander angeordnete Weichgewebe-Koagulationselektroden (294), die auf einer Elektroden-Halteanordnung gehalten sind, die in einer Abstandsrelation zum Griff angeordnet ist, wobei die jeweilige Größe und der Abstand von den mindestens zwei Weichgewebe-Koagulationselektroden (294) derart ist, dass dadurch gleichzeitiges Übertragen von Energie zu einer indifferenten Elektrode ein Gebiet von koaguliertem Gewebe produziert, das sich über die mindestens zwei Weichgewebe-Koagulationselektroden erstreckt, **gekennzeichnet durch** eine relativ kurze geschmeidige Welle (310), die ein mit dem Griff (312) zusammenhängendes Proximalende und ein mit der Elektroden-Halteanordnung verbundenes Distalende hat, wobei die geschmeidige Welle einen Biegemodul von zwischen ungefähr 3 lb.-in.$^2$ (86 N-cm$^2$) und ungefähr 50 lb.-in.$^2$ (1435 N-cm$^2$) hat, wobei der Biegemodul ein Produkt aus dem Elastizitätsmodul und dem Trägheitsmoment der Welle (310) ist.

2. Weichgewebe-Koagulationssonde gemäß Anspruch 1, ferner aufweisend:

einen Temperatursensor, der sich am Rand des Längsendes von mindestens einer der Weichgewebe-Koagulationselektroden befindet.

3. Weichgewebe-Koagulationssonde gemäß Anspruch 1, ferner aufweisend:

einen Temperatursensor, der mindestens einer der Weichgewebe-Koagulationselektroden zugeordnet ist.

4. Weichgewebe-Koagulationssonde gemäß Anspruch 1, wobei die Elektroden-Halteanordnung an ihrem Distalende eine biegbare Splineanordnung (256) aufweist, die eine vorherbestimmte Konfiguration hat, wobei die Splineanordnung dahingehend eingerichtet ist, als Antwort auf ein Anlegen einer externen Kraft zu kollabieren und als Antwort auf eine Rücknahme der externen Kraft zu der vorherbestimmten Konfiguration zu expandieren, wobei die Weichgewebe-Koagulationselektroden (294) auf der biegbaren Splineanordnung angeordnet sind.

5. Weichgewebe-Koagulationssonde gemäß Anspruch 4, ferner aufweisend:

ein im Wesentlichen röhrenförmiges Teil (264), das um die Welle (254) herum positioniert ist und relativ zur Welle verschiebbar ist;

wobei die Splineanordnung (256) dahingehend eingerichtet ist, als Antwort auf eine Bewegung des im Wesentlichen röhrenförmigen Teils darüber zu kollabieren und als Antwort auf ein Zurückziehen des im Wesentlichen röhrenförmigen Teils zu expandieren.

6. Weichgewebe-Koagulationssonde gemäß Anspruch 4, wobei die biegbare Splineanordnung (256) einen im Wesentlichen ringförmigen Anteil (262) aufweist.

7. Weichgewebe-Koagulationssonde gemäß Anspruch 6, wobei die Welle (254) eine Längsachse definiert und der im Wesentlichen ringförmige Anteil (262) in einer Ebene liegt, die im Wesentlichen senkrecht zu der Längsachse orientiert ist.

8. Weichgewebe-Koagulationssonde gemäß Anspruch 4, wobei die biegbare Splineanordnung eine im Wesentlichen dreiecksförmige Splineanordnung (272) aufweist.

9. Weichgewebe-Koagulationssonde gemäß Anspruch 8, wobei die im Wesentlichen dreiecksförmige Splineanordnung (272) einen Distalschenkel (278) aufweist, der ein erstes und ein zweites Ende definiert, wobei der Distalschenkel vom ersten Ende zum zweiten Ende nichtlinear ist.

10. Weichgewebe-Koagulationssonde gemäß Anspruch 6, wobei die biegbare Splineanordnung die Form einer Schleife (300) hat.

**11.** Weichgewebe-Koagulationssonde gemäß Anspruch 1, wobei die Elektroden-Halteanordnung eine distale Endanordnung (350) aufweist, die ein Distalteil aufweist, das entweder flexibel, ziemlich flexibel oder geschmeidig ist, wobei die Weichgewebe-Koagulationselektroden (294) auf dem Distalteil angeordnet sind.

**12.** Weichgewebe-Koagulationssonde gemäß Anspruch 11, ferner aufweisend:

ein röhrenförmiges Teil (354), das um einen vorbestimmten Abschnitt der Welle herum positioniert ist und relativ dazu bewegbar ist, wobei das röhrenförmige Teil ein Proximalende, ein Distalende und eine Öffnung zwischen dem Distalende und dem Proximalende definiert;

wobei die distale Endanordnung aufweist eine Schwenkanordnung (360) mit einem ersten Schwenkteil (364), das mit einem Distalabschnitt des Distalteils verbunden ist, und einem zweiten Schwenkteil (362), das mit dem röhrenförmigen Teil verbunden ist.

**13.** Weichgewebe-Koagulationssonde gemäß Anspruch 1, wobei der Griff (312") eine Energie-Steuervorrichtung (315) aufweist, die zum selektiven Steuern der Energieübertragung von einer Energiequelle zu den mindestens zwei Weichgewebe-Koagulationselektroden (294) eingerichtet ist.

**14.** Weichgewebe-Koagulationssonde gemäß Anspruch 13, wobei die Energie-Steuervorrichtung (315) einen Ein-Aus-Schalter aufweist.

**15.** Weichgewebe-Koagulationssonde gemäß Anspruch 13, wobei die Energie-Steuervorrichtung (315) mindestens zwei Energie-Steuervorrichtungen aufweist, die jeweils mit den mindestens zwei Weichgewebe-Koagulationselektroden (294) gekoppelt sind.

**16.** Weichgewebe-Koagulationssonde gemäß Anspruch 15, ferner aufweisend:

mindestens zwei Anzeigevorrichtungen (317), die zum Anzeigen des jeweiligen Status der mindestens zwei Weichgewebe-Koagulationselektroden (294) eingerichtet sind.

**17.** Weichgewebe-Koagulationssonde gemäß Anspruch 15, ferner aufweisend:

eine globale Energie-Steuervorrichtung (313), die zum selektiven Steuern der Energieübertragung von der Energiequelle zu jeder der Weichgewebe-Koagulationselektroden (294) eingerichtet ist.

**18.** Weichgewebe-Koagulationssonde gemäß Anspruch 13, ferner aufweisend:

eine Anzeigevorrichtung (317), die zum Anzeigen des Status der Energie-Steuervorrichtung eingerichtet ist.

**19.** Weichgewebe-Koagulationssonde gemäß Anspruch 1, ferner aufweisend:

ein Verbindungsteil (319), das zum lösbaren Verbinden des Griffs mit der Welle eingerichtet ist.

**20.** Weichgewebe-Koagulationssonde gemäß Anspruch 1, ferner aufweisend:

eine Druckanlege-Sonde (650), die einen länglichen Hauptkörper-Abschnitt (652) und eine Eingriff-Vorrichtung (654) aufweist, die zum lösbaren Im-Eingriff-stehen der Welle eingerichtet ist.

**21.** Weichgewebe-Koagulationssonde gemäß Anspruch 20, wobei die Eingriff-Vorrichtung (654) ein im Allgemeinen C-förmiges Teil aufweist.

**22.** Weichgewebe-Koagulationssonde gemäß Anspruch 1, ferner aufweisend:

eine Kupplungsvorrichtung (664), aufweisend ein Basisteil (666), das derart eingerichtet ist, entfernbar an einem ersten Abschnitt der Welle befestigt zu sein, und eine Eingriff-Vorrichtung (668), die mit dem Basisteil verbunden ist und derart eingerichtet ist, entfernbar an mit einem zweiten Abschnitt der Welle befestigt zu sein;

wobei die Welle eine Schleife definiert, wenn der erste Abschnitt an dem Basisteil befestigt ist und der zweite Abschnitt an der Kupplungsvorrichtung befestigt ist.

**Revendications**

**1.** Sonde (308) de coagulation pour un tissu mou comprenant une poignée (312) et au moins deux électrodes espacées (294) de coagulation pour un tissu mou supportées sur un ensemble de support d'électrodes espacé par rapport à la poignée, la taille respective et l'espacement des au moins deux électrodes (294) de coagulation pour tissu mou étant tels qu'une transmission simultanée d'énergie envoyée de ce fait à une électrode indifférente produit une zone de tissu coagulé, qui s'étend sur au moins deux électrodes de coagulation du tissu mou, **caractérisée par**

une tige malléable relativement courte (310) possédant une extrémité proximale associée à la poignée (312) et une extrémité distale raccordée à l'ensemble de support d'électrodes, ladite tige malléable possédant un module de flexion compris entre environ 3 livre.pouce$^2$ (86 N.cm$^2$) et environ 50 livre.pouce$^2$ (1435 N.cm$^2$), le module de flexion étant le produit du module d'élasticité par le moment d'inertie de la tige (310).

2. Sonde de coagulation pour tissu mou selon la revendication 1, comprenant en outre:

un capteur de température situé au niveau du bord d'extrémité longitudinal d'au moins l'une des électrodes de coagulation pour tissu mou.

3. Sonde de coagulation pour tissu mou selon la revendication 1, comprenant en outre:

un capteur de température associé à au moins l'une des électrodes de coagulation pour tissu mou.

4. Sonde de coagulation pour tissu mou selon la revendication 1, dans laquelle l'ensemble formant support d'électrodes comprend un ensemble à cannelures (256) pouvant être coudées, au niveau de son extrémité distale possédant une configuration prédéterminée, l'ensemble à cannelures étant adapté pour s'aplatir en réponse à une application d'une force externe et pour s'étendre à la configuration prédéterminée en réponse à une suppression de la force externe, lesdites électrodes (294) de coagulation pour tissu mou étant situées sur l'ensemble à cannelures pouvant être coudé.

5. Sonde de coagulation pour tissu mou selon la revendication 4, comprenant en outre:

un élément essentiellement tubulaire (264) positionné autour de la tige (254) et pouvant glisser par rapport à la tige;

dans laquelle l'ensemble à cannelures (256) est adapté pour s'aplatir en réponse au déplacement de l'élément essentiellement tubulaire au-dessus de cet ensemble et s'étendre en prenant la configuration prédéterminée en réponse à un retrait de l'élément essentiellement tubulaire.

6. Sonde de coagulation pour tissu mou selon la revendication 4, dans laquelle l'ensemble à cannelures (256) pouvant être coudé inclut une partie essentiellement annulaire (262).

7. Sonde de coagulation pour tissu mou selon la revendication 6, dans laquelle la tige (354) définit un axe longitudinal et la partie essentiellement annulaire (262) est située dans un plan orienté essentiellement perpendiculairement à l'axe longitudinal.

8. Sonde de coagulation pour tissu mou selon la revendication 4, dans laquelle l'ensemble à cannelures pouvant être coudé comprend un ensemble à cannelures de forme essentiellement triangulaire (272).

9. Sonde de coagulation pour tissu mou selon la revendication 8, dans laquelle l'ensemble à cannelures de forme essentiellement triangulaire (272) comprend une branche distale (278) définissant des première et seconde extrémités, la branche distale s'étendant d'une manière non linéaire depuis la première extrémité jusqu'à la seconde extrémité.

10. Sonde de coagulation pour tissu mou selon la revendication 6, dans laquelle l'ensemble à cannelures pouvant être coudé se présente sous la forme d'une boucle (300).

11. Sonde de coagulation pour tissu mou selon la revendication 1, dans laquelle l'ensemble formant support d'électrodes comprend un ensemble de pointe distale (350) incluant un élément distal qui est soit flexible, soit assez flexible, soit malléable, les électrodes (294) de coagulation pour tissu mou étant situées sur l'élément distal.

12. Sonde de coagulation pour tissu mou selon la revendication 11, comprenant en outre:

un élément tubulaire (354) disposé autour d'une partie prédéterminée de l'arbre et déplaçable par rapport à ce dernier, l'élément tubulaire définissant une extrémité proximale, une extrémité distale et une ouverture entre les extrémités distale et proximale;

dans laquelle l'ensemble de pointe distale inclut un ensemble formant pivot (360) possédant un premier élément formant pivot (364) raccordé à une extrémité distale de l'élément distal et un second élément formant pivot (362) raccordé à l'élément tubulaire.

13. Sonde de coagulation pour tissu mou selon la revendication 1, dans laquelle la poignée (312") inclut un dispositif de commande d'énergie (315) adapté pour commander sélectivement la transmission d'énergie depuis une source d'énergie aux au moins deux électrodes (294) de coagulation pour tissu mou.

**14.** Sonde de coagulation pour tissu mou selon la revendication 13, dans laquelle le dispositif de commande d'énergie (315) comprend un interrupteur marche-arrêt.

**15.** Sonde de coagulation pour tissu mou selon la revendication 13, dans laquelle le dispositif de commande d'énergie (315) comprend au moins deux dispositifs de commande d'énergie couplés respectivement aux au moins deux électrodes (294) de coagulation pour tissu mou.

**16.** Sonde de coagulation pour tissu mou selon la revendication 15, comprenant en outre:

au moins deux indicateurs (317) adaptés pour indiquer les états respectifs des au moins deux électrodes (294) de coagulation pour tissu mou.

**17.** Sonde de coagulation pour tissu mou selon la revendication 15, comprenant en outre:

un dispositif (313) de commande d'énergie global adapté pour commander sélectivement la transmission d'énergie depuis la source d'énergie vers chacune des électrodes (294) de coagulation pour tissu mou.

**18.** Sonde de coagulation pour tissu mou selon la revendication 13, comprenant en outre:

un indicateur (317) adapté pour indiquer l'état du dispositif de commande d'énergie.

**19.** Sonde de coagulation pour tissu mou selon la revendication 1, comprenant en outre:

un raccord (319) adapté pour raccorder de façon amovible la poignée à la tige.

**20.** Sonde de coagulation pour tissu mou selon la revendication 1, comprenant en outre:

une sonde d'application de pression (650) incluant une partie allongée formant corps principal (652), un dispositif d'engagement (654) adapté pour coopérer de façon amovible avec la tige.

**21.** Sonde de coagulation pour tissu mou selon la revendication 20, dans laquelle le dispositif d'engagement (654) comprend un élément en forme générale de C.

**22.** Sonde de coagulation pour tissu mou selon la revendication 1, comprenant en outre:

un dispositif de couplage (664) comprenant un élément de base (666) adapté pour être fixé de façon amovible à une première partie de la tige et un dispositif d'engagement (668) raccordé à l'élément de base et adapté pour être fixé de façon amovible à une seconde partie de la tige; l'arbre définissant une boucle lorsque la première partie est fixée à l'élément de base et que la seconde partie est fixée au dispositif de couplage.

*FIG. 2*

*FIG. 1*

*FIG. 3A*

EP 1 024 761 B1

FIG. 3B

FIG. 4

FIG. 5

PULMONARY VEIN

EP 1 024 761 B1

6b

254

264′

FIG. 6A

292

6b

254

264′

*FIG. 6B*

292

294

299

*FIG. 7*

295

294′

297

280

*FIG. 8A*

294

296

*FIG. 8B*

FIG. 9A

FIG. 9B

FIG. 9C

EP 1 024 761 B1

FIG. 9D

FIG. 9E

FIG. 9F

FIG. 10A

FIG. 10B

FIG. 10C

EP 1 024 761 B1

FIG. 10D

FIG. 10E

EP 1 024 761 B1

FIG. 10F

FIG. 10G

312''

323

10G

310

312'''

319

321

EP 1 024 761 B1

37

FIG. 10H

308

325

327B

327a

329a

329b

SELECTED  7 6 5 4 3 2 1

POWER CONTROL
UNIT

① ② ③ ④ ⑤ ⑥ ⑦

ENERGY
SOURCE

EP 1 024 761 B1

FIG. 10I

FIG. 11A

FIG. 11B

FIG. 11C

FIG. 12

FIG. 13

EP 1 024 761 B1

FIG. 14

FIG. 15

## FIG. 16

## FIG. 18

## FIG. 17

## FIG. 19

EP 1 024 761 B1

FIG. 20

FIG. 21

418

252

422

294

426

428

420

424

416

44

EP 1 024 761 B1

FIG. 22

EP 1 024 761 B1

432′

*FIG. 24A*

432″

θ

*FIG. 24B*

432

436

*FIG. 23*

FIG. 25

FIG. 26

FIG. 27

452

454

ATRIAL
APPENDAGE

ESTABLISH COLLECTIVE
DUTYCYCLE E(J)
SELECT AMP E(J)
ESTABLISHED TEMP$_{SET}$

DELIVER PULSE TO EACH ELECTRODE REGION E(J)
P. E(J)~AMP E(J)$^2$ x DUTYCYCLE (J)

J = 1 TO N
SAMPLE
TEMP (J)

REDUCE
AMP E(J)

PROCESS
TEMP(J)≈TEMP$_{SET}$

INCREASE
AMP E(J)

MAINTAIN
AMP E(J)

FIG. 30

EP 1 024 761 B1

FIG. 28

EP 1 024 761 B1

TO ISOLATED OUTPUT STAGE 516

FROM ELECTRODES 501, 502, 503

514

536

534

538

533

535

537

513

539

540

541

542

543

15V

DGND

−5V

ESEL

TO MICROCONTROLLER 531

FIG. 29

# FIG. 31

600

$W^1(1,3)$

N(1,3)

$W^1(2,3)$

TS1(n)

$W^1(1,2)$

$W^2(3,1)$

$W^2(2,1)$

N(1,2)

$T_{MAXPRED}(t)$

$W^1(2,2)$

$W^2(1,1)$

TS2(n)

$W^1(1,1)$

N(1,1)

$W^1(2,1)$

FIG. 33

FIG. 32

FIG. 34

FIG. 35

FIG. 36

FIG. 37

660

662

664

666

670

668

EP 1 024 761 B1

FIG. 38

EP 1 024 761 B1

FIG. 39

FIG. 40

308

314

674b

664

674a

676

670

666

664

EP 1 024 761 B1